(19) **Europäisches Patentamt / European Patent Office / Office européen des brevets**

(11) **EP 1 700 119 B1**

(12) **EUROPEAN PATENT SPECIFICATION**

(45) Date of publication and mention
of the grant of the patent:
**10.02.2010 Bulletin 2010/06**

(21) Application number: **04813979.4**

(22) Date of filing: **14.12.2004**

(51) Int Cl.:
*G01N 33/50* (2006.01)  *G01N 33/53* (2006.01)
*C07K 7/08* (2006.01)  *C12N 9/86* (2006.01)
*C12N 5/10* (2006.01)  *C07K 16/12* (2006.01)

(86) International application number:
**PCT/US2004/041736**

(87) International publication number:
**WO 2005/062042 (07.07.2005 Gazette 2005/27)**

(54) **BETA-LACTAMASE CD4+ T-CELL EPITOPES**

BETA-LACTAMASE-CD4+-T-ZELLEPITOPE

EPITOPES LYMPHOCYTES T BETA-LACTAMASE CD4+

(84) Designated Contracting States:
**AT BE BG CH CY CZ DE DK EE ES FI FR GB GR HU IE IS IT LI LT LU MC NL PL PT RO SE SI SK TR**

(30) Priority: **18.12.2003 US 531128 P**

(43) Date of publication of application:
**13.09.2006 Bulletin 2006/37**

(73) Proprietor: **GENENCOR INTERNATIONAL, INC.**
**Palo Alto, California 94304 (US)**

(72) Inventors:
 • **HARDING, Fiona, A.**
 **Santa Clara, CA 95050 (US)**
 • **SCHELLENBERGER, Volker**
 **Palo Alto, CA 94303 (US)**

(74) Representative: **Forrest, Graham Robert et al**
**Mewburn Ellis LLP**
**33 Gutter Lane**
**London**
**EC2V 8AS (GB)**

(56) References cited:
 **WO-A-01/90346      WO-A-02/47717**
 **WO-A-02/069232      WO-A-03/072746**

• **BARNAUD GUILENE ET AL: "Extension of resistance to cefepime and cefpirome associated to a six amino acid deletion in the H-10 helix of the cephalosporinase of an Enterobacter cloacae clinical isolate"** FEMS MICROBIOLOGY LETTERS, vol. 195, no. 2, 20 February 2001 (2001-02-20), pages 185-190, XP002326992 ISSN: 0378-1097
• **DATABASE EMBL [Online] 14 September 2000 (2000-09-14), "Enterobacter cloacae ampR gene for transcriptional activator and ampC gene for class C beta-lactamase, strain CHE"** XP002326994 retrieved from EBI accession no. EM_PRO:AJ278994 Database accession no. AJ278994
• **DATABASE EMBL [Online] 24 January 2002 (2002-01-24), "Enterobacter dissolvens partial ampC gene for beta-lactamase"** XP002326995 retrieved from EBI accession no. EM_PRO: AJ311363 Database accession no. AJ311363
• **DATABASE EMBL [Online] 23 March 2003 (2003-03-23), "Yokenella regensburgei beta-lactamase (ampC) gene, partial cds."** XP002326996 retrieved from EBI accession no. EM_PRO:AY221960 Database accession no. AY221960
• **DATABASE EMBL [Online] 11 April 2002 (2002-04-11), "Escherichia fergusonii AmpC (ampC) gene, complete cds."** XP002326997 retrieved from EBI accession no. EM_PRO: AF492449 Database accession no. AF492449

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **OLSSON O ET AL: "AMP-C BETA LACTAMASE HYPER PRODUCTION IN ESCHERICHIA-COLI NATURAL AMPICILLIN RESISTANCE GENERATED BY HORIZONTAL CHROMOSOMAL DNA TRANSFER FROM SHIGELLA-SONNEI"** PROCEEDINGS OF THE NATIONAL ACADEMY OF SCIENCES OF THE UNITED STATES OF AMERICA, vol. 80, no. 24, 1983, pages 7556-7560, XP002326993 ISSN: 0027-8424

**Description**

## FIELD OF THE INVENTION

The present invention provides beta-lactamase CD4+ T-cell epitopes, as well as novel variants that exhibit reduced immunogenic responses, as compared to the parental beta-lactamase. The present invention further provides DNA molecules that encode novel beta-lactamase variants, and host cells comprising DNA encoding novel beta-lactamase variants, as well as methods for making beta-lactamases less immunogenic. In addition, the present invention provides various compositions that comprise these beta-lactamase variants that are less immunogenic than the wild-type beta-lactamases.

## BACKGROUND OF THE INVENTION

Beta-lactamases (β-lactamases) are enzymes that catalyze the hydrolysis of beta-lactams. These enzymes are clinically important because a beta-lactam group is present in many commonly used antimicrobials, including penicillin, cephalosporin, and their derivatives. Beta-lactam antimicrobials inhibit enzymes that are involved in the synthesis of bacterial cell walls. As resistance to beta-lactams confers a strong survival advantage to bacteria, development of beta-lactam resistance through the production of enzymes such as beta-lactamases provides an important survival mechanism. Beta-lactamases are structurally related to and may be evolved from enzymes that are involved in cell wall synthesis (*i.e.*, penicillin-binding proteins).

More than 190 beta-lactamases are known. These enzymes have been identified on plasmids and in chromosomes/genomes, in which their expression is either constitutive or inducible. Beta-lactamases vary widely in their efficacy against antimicrobials. For example, some are highly active against penicillins, while others are more active against cephalosporins, and still others are efficacious against both. Thus, the production of beta-lactamases by pathogenic organisms represents a significant obstacle in the treatment of some infections.

WO 2002/047717 discusses the use of beta-lactamases in enzyme-prodrug therapy and provides means for targeting beta-lactamases to sites of interest.

In addition to their characteristic inactivation of beta-lactams, beta-lactamases are immunogenic. Thus, in many individuals, upon exposure to these enzymes, an immune response is mounted against epitopes in the enzymes. While some studies have provided methods of reducing the allergenicity/immunogenicity of certain proteins and identification of epitopes which cause allergic or other immunogenic reactions in some individuals, the assays used to identify these epitopes generally involve measurement of IgE and IgG in the sera of those who have been previously exposed to the protein. However, once an Ig reaction has been initiated, sensitization has already occurred. Accordingly, there is a need to identify and/or produce proteins for use in humans and other animals, which produce an a reduced immunologic response.

## SUMMARY OF THE INVENTION

The present invention provides beta-lactamase CD4+ T-cell epitopes, as well as novel variants that exhibit reduced immunogenic responses, as compared to the parental beta-lactamase. The present invention further provides DNA molecules that encode novel beta-lactamase variants, and host cells comprising DNA encoding novel beta-lactamase variants, as well as methods for making beta-lactamases less immunogenic. In addition, the present invention provides various compositions that comprise these beta-lactamase variants that are less immunogenic than the wild-type beta-lactamases. In some specific embodiments, the present invention provides beta-lactamase variants with reduced immunogenicity that are identified and/or characterized using the methods of the present invention. The parental beta-lactamase is from Enterobacter cloacae.

In particular, the invention provides an isolated variant microbial beta-lactamase, which is a variant of a precursor beta-lactamase from *Enterobacter cloacae,* the variant having an amino acid sequence comprising at least one modification of an amino acid made at a position in the precursor which corresponds in position in primary or tertiary structure to a position in an epitope defined by SEQ ID NO : 2, SEQ ID NO: 3, SEQ ID NO: 4 or SEQ ID NO: 5 of *E. cloacae* beta-lactamase having the amino acid sequence of SEQ ID NO: 1, whereby the immunogenic response produced by said variant beta-lactamase is less than the immunogenic response produced by said precursor beta-lactamase, wherein said variant beta-lactamase has at least 80% or at least 90% amino acid sequence identity to said precursor beta-lactamase.

The invention also provides a method of reducing immunogenicity of a precursor microbial beta-lactamase from *Enterobacter cloacae*, comprising modifying at least one amino acid at a position which corresponds in position in primary or tertiary structure to a position in an epitope defined by SEQ ID NO : 2, SEQ ID NO: 3, SEQ ID NO: 4 or SEQ ID NO: 5 of *E. cloacae* beta-lactamase having the amino acid sequence of SEQ ID NO: 1 to produce a variant beta-lactamase, whereby said variant beta-lactamase is less immunogenic than the precursor beta-lactamase.

The precursor beta-lactamase may be modified by addition, deletion or substitution of at least one amino acid in said T cell epitope.

The precursor beta-lactamase may be modified by substituting the amino acid sequence of said T-cell epitope with an analogous sequence from a homolog of said precursor beta-lactamase, wherein said substitution mimics the tertiary structure of said T-cell epitope.

The variant beta-lactamase may comprise at least one epitope comprising an amino acid sequence selected from the group consisting of SEQ ID NOS: 10, 11, 20, 21, 25, 40, 48, 49, 50, 52, 53, 59, 69 and 84.

In some embodiments, the beta-lactamase comprises at least a portion of the sequence set forth in SEQ ID NO:1.

The present invention also provides methods for reducing the immunogenicity of beta-lactamase from Enterobacter cloacae comprising the steps of: (a) identifying at least one T-cell epitope in the beta-lactamase by (i) contacting an adherent monocyte-derived dendritic cell that has been differentiated by exposure to at least one cytokine *in vitro*, with at least one peptide comprising the T-cell epitope; and (ii) contacting the dendritic cell and the peptide with a naive T-cell, wherein the naïve T-cell has been obtained from the same source as the adherent monocyte-derived dendritic cell, and whereby the T-cell proliferates in response to the peptide; and (b) modifying the beta-lactamase to neutralize the T-cell epitope to produce a variant beta-lactamase, such that the variant beta-lactamase induces less than or substantially equal to the baseline proliferation of the naive T-cells;
wherein said epitope of the beta-lactamase is modified by substituting the amino acid sequence of the T-cell epitope with an analogous sequence from a homolog of the beta-lactamase, wherein the substitution substantially mimics the tertiary structure of the T-cell epitope, and wherein said epitope is selected from the group consisting of SEQ ID NOS: 2, 3, 4, and 5. The present invention further provides beta-lactamases produced using the above method and as defined in the claims.

The variant beta-lactamases may comprise at least a portion of the beta lactamase sequence set forth in SEQ ID NO:1. The present invention further provides expression vectors comprising nucleic acid sequences encoding a variant beta-lactamase of the invention. The present invention also provides host cells comprising at least one expression vector comprising nucleic acid sequences encoding a variant beta-lactamase of the invention. The present invention further provides the beta-lactamase(s) produced using these host cells

The present invention also provides variant beta-lactamases, as defined in the claims, comprising at least one alteration in at least one epitope comprising an amino acid sequence selected from the group consisting of SEQ ID NOS:10, 11, 20, 21, 25, 40, 48, 49, 50, 52, 53, 59, 69, and 84. In some embodiments, the present invention provides beta lactamases comprising at least one epitope comprising an amino acid sequence selected from the group consisting of SEQ ID NOS:55-90. The immunogenic response produced by the variant beta-lactamase is less than the immunogenic response produced by the precursor beta-lactamase.

The present invention further provides compositions comprising nucleic acids sequences encoding variant beta-lactamases of the invention, as well as expression vectors that comprise the nucleic acid, and host cells transformed with the expression vectors. In some embodiments, the present invention provides peptides comprising at least one amino acid sequence selected from the group consisting of SEQ ID NOS:10, 11, 20, 21, 25, 40, 48, 49, 50, 52, 53, 59, 69, and 84. In some embodiments, the present invention provides beta lactamases comprising at least one epitope comprising an amino acid sequence selected from the group consisting of SEQ ID NOS: 10, 11, 20, 21, 25, 40, 48, 49, 50, 52, 53, 59, 69 and 84.

The present invention still further provides pharmaceutical and consumer-related products, including, but not limited to such compositions as compositions that comprise the variant beta-lactamases of the present invention. In some embodiments, the beta-lactamase variants find use in compositions such as those used to treat abnormal cells (*e.g.*, cancer cells). For example, the present invention finds use as an enzyme suitable for converting prodrugs (*i.e.*, non-toxic compounds) into cytotoxic compounds. Thus, the present invention finds use in such applications as antibody-directed enzyme prodrug therapy, as well as targeted therapy that does not rely upon the use of antibodies and/or antibody components. Indeed, the present invention provides variant beta-lactamases suitable for use in various applications, settings, and compositions.

## DESCRIPTION OF THE FIGURES

Figure 1 provides a table showing the responses of 69 community donors to a peptide set describing the amino acid sequence of beta-lactamase.
Figure 2 provides a graph showing the responses to peptide #6 (SEQ ID NO:2) and two variants (SEQ ID NOS:10 and 11).
Figure 3 provides a graph showing the responses to peptide #36 (SEQ ID NO:3) and three variants (SEQ ID NOS:

20, 21, and 25).

Figure 4 provides a graph showing the responses to peptide #49 (SEQ ID NO:4) and one variant (SEQ ID NO:40).

Figure 5 provides a graph showing the responses to peptide #107 (SEQ ID NO:5), and five variants (SEQ ID NOS: 48, 49, 50, 52, and 53).

Figure 6 provides a graph showing the responses to peptide #49 (SEQ ID NO:4) and a series of modified epitopes.

Figure 7 provides a graph showing the responses to peptide #49 with the substitution I155F (SEQ ID NO:59) and a pepset based on this sequence.

Figure 8 provides a graph showing the responses to peptide #49 (SEQ ID NO:4) with the substitution I155V (SEQ ID NO:63) and a pepset based on this sequence.

Figure 9 provides a graph showing the responses to peptide #49 (SEQ ID NO:4) with the substitution I155L (SEQ ID NO:69) and a pepset based on this sequence.

Figure 10 provides a graph showing the responses to peptide #49 (SEQ ID NO:4) with the substitution T147Q (SEQ ID NO:75) and a pepset based on this sequence.

Figure 11 provides a graph showing the responses to peptide #49 (SEQ ID NO:4) with the substitution L149S (SEQ ID NO:82) and a pepset based on this sequence.

Figure 12 provides a graph showing the responses to peptide #49 (SEQ ID NO:4) with the substitution L149R (SEQ ID NO:87) and a pepset based on this sequence.

Figure 13 provides graphs showing the results from the PBMC assay used to test beta-lactamase (SEQ ID NO:1) and two epitope-modified beta-lactamases. Panel A is a graph showing the average proliferative responses obtained for each enzyme, while Panel B is a graph showing the percent of responders for each enzyme.

Figure 14 provides graphs showing the proliferative response of mouse splenocytes from BLA (Panel A) or CD1.1 (Panel B) immunized mice to BLA peptides.

Figure 15 provides graphs showing the results for splenocytes from immunized mice tested *in vitro* for their proliferative response to whole protein BLA (Panel A) or CD1.1 (Panel B). The results are shown in Figure 15.

Figure 16 provides graphs showing ELISA results for the thrice-immunized mice.

## DESCRIPTION OF THE INVENTION

The present invention provides beta-lactamase variants, as defined in the claims, that exhibit reduced immunogenic responses, as compared to the parental beta-lactamase. The present invention further provides DNA molecules that encode said beta-lactamase variants, and host cells comprising DNA encoding said beta-lactamase variants, as well as methods for making beta-lactamases less immunogenic. In addition, the present invention provides various compositions that comprise these beta-lactamase variants that are less immunogenic than the wild-type beta-lactamases. In some specific embodiments, the present invention provides beta-lactamase variants with reduced immunogenicity that are identified and/or characterized using the methods of the present invention.

### Definitions

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionaries of many of the terms used in the invention. Although any methods and materials similar or equivalent to those described herein find use in the practice of the present invention, the preferred methods and materials are described herein. Accordingly, the terms defined immediately below are more fully described-by reference to the Specification as a whole. Also, as used herein, the singular "a", "an" and "the" includes the plural reference unless the context clearly indicates otherwise.

As used herein, "beta-lactamase" refers to any enzyme that breaks down beta lactam rings (*i.e.*, a heteroatomic ring structure comprising three carbon atoms and one nitrogen atom). Beta lactam rings are components of several antimicrobials, including but not limited to penicillin, cephalosporin, and other related compounds. In some preferred embodiments, the beta-lactamase of the present invention is native enzyme, while in some other preferred embodiments, the beta-lactamase of the present invention is recombinant. In some additionally preferred embodiments, the beta-lactamases of the present invention are modified, such that the beta-lactamases stimulate a lower immunogenic reaction than native beta-lactamases.

As used herein, the term "recombinant oligonucleotide" refers to an oligonucleotide created using molecular biological manipulations, including but not limited to, the ligation of two or more oligonucleotide sequences generated by restriction enzyme digestion of a polynucleotide sequence, the synthesis of oligonucleotides (*e.g.*, the synthesis of primers or oligonucleotides) and the like.

As used herein, "recombinant beta-lactamase" refers to an beta-lactamase in which the DNA sequence encoding beta-lactamase is modified to produce a variant (or mutant) DNA sequence which encodes the substitution, deletion or insertion of one or more amino acids in the naturally-occurring amino acid sequence. Suitable methods to produce such modification are well-known to those in the art.

The term "recombinant DNA molecule" as used herein refers to a DNA molecule that is comprised of segments of DNA joined together by means of molecular biological techniques.

As used herein, the term "enzymatic conversion" refers to the modification of a carbon substrate to an intermediate or the modification of an intermediate to an end-product by contacting the substrate or intermediate with an enzyme. In some embodiments, contact is made by directly exposing the substrate or intermediate to the appropriate enzyme. In other embodiments, contacting comprises exposing the substrate or intermediate to an organism that expresses and/or excretes the enzyme, and/or metabolizes the desired substrate and/or intermediate to the desired intermediate and/or end-product, respectively.

As used herein, "wild-type" and "native" proteins are those found in nature. The terms "wild-type sequence," and "wild-type gene" are used interchangeably herein, to refer to a sequence that is native or naturally occurring in a host cell. In some embodiments, the wild-type sequence refers to a sequence of interest that is the starting point of a protein engineering project. The genes encoding the naturally-occurring (*i.e.*, precursor) protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

The term "sample" as used herein is used in its broadest sense. However, in preferred embodiments, the term is used in reference to a sample (*e.g.*, an aliquot) that comprises a peptide (*i.e.*, a peptide within a pepset, that comprises a sequence of a protein of interest) that is being analyzed, identified, modified, and/or compared with other peptides. Thus, in most cases, this term is used in reference to material that includes a protein or peptide that is of interest.

As used herein, "Stimulation Index" (SI) refers to a measure of the T-cell proliferative response of a peptide compared to a control. The SI is calculated by dividing the average CPM (counts per minute) obtained in testing the CD4$^+$ T-cell and dendritic cell culture containing a peptide by the average CPM of the control culture containing dendritic cells and CD4$^+$ T-cells but without the peptides. This value is calculated for each donor and for each peptide. While SI values of between about 1.5 to 4.5 may be used to indicate a positive response, the preferred SI value to indicate a positive response is between 2.5 and 3.5, inclusive, preferably between 2.7 and 3.2 inclusive, and more preferably between 2.9 and 3.1 inclusive. The most preferred embodiments described herein use a SI value of 2.95.

As used herein, the term "dataset" as used herein refers to compiled data for a set of peptides and a set of donors for each protein.

As used herein, the term "pepset" refers to the set of peptides obtained from each test protein (*i.e.*, protein of interest). These peptides in the pepset (or "peptide sets") are tested with cells from each donor.

As used herein, the terms "purified" and "isolated" refer to the removal of contaminants from a sample. For example, beta-lactamases are purified by removal of contaminating proteins and other compounds within a solution or preparation that are not beta-lactamases. In some embodiments, recombinant beta-lactamases are expressed in bacterial host cells and these recombinant beta-lactamases are purified by the removal of other host cell constituents; the percent of recombinant beta-lactamase polypeptides is thereby increased in the sample.

As used herein, "background level" and "background response" refer to the average percent of responders to any given peptide in the dataset for any tested protein. This value is determined by averaging the percent responders for all peptides in the set, as compiled for all the tested donors.

As an example, a 3% background response would indicate that on average there would be three positive (SI greater than 2.95) responses for any peptide in a dataset when tested on 100 donors.

As used herein, "antigen presenting cell" ("APC") refers to a cell of the immune system that presents antigen on its surface, such that the antigen is recognizable by receptors on the surface of T-cells. Antigen presenting cells include, but are not limited to dendritic cells, interdigitating cells, activated B-cells and macrophages.

The term "lymphoid"-when used in reference to a cell line or a cell, means that the cell line or cell is derived from the lymphoid lineage and includes cells of both the B and the T lymphocyte lineages.

As used herein, the terms "T lymphocyte" and "T-cell," encompass any cell within the T lymphocyte lineage from T-cell precursors (including Thy1 positive cells which have not rearranged the T cell receptor genes) to mature T cells (*i.e.*, single positive for either CD4 or CD8, surface TCR positive cells).

As used herein, the terms "B lymphocyte" and "B-cell" encompasses any cell within the B-cell lineage from B-cell precursors, such as pre-B-cells (B220$^+$ cells which have begun to rearrange Ig heavy chain genes), to mature B-cells and plasma cells.

As used herein, "CD4$^+$ T-cell" and "CD4 T-cell" refer to helper T-cells, while "CD8$^+$ T-cell" and CD8 T-cell" refer to cytotoxic T-cells.

As used herein, "B-cell proliferation," refers to the number of B-cells produced during the incubation of B-cells with the antigen presenting cells, with or without antigen.

As used herein, "baseline B-cell proliferation," as used herein, refers to the degree of B-cell proliferation that is normally seen in an individual in response to exposure to antigen presenting cells in the absence of peptide or protein antigen. For the purposes herein, the baseline B-cell proliferation level is determined on a per sample basis for each individual as the proliferation of B-cells in the absence of antigen.

As used herein, "B-cell epitope," refers to a feature of a peptide or protein that is recognized by a B-cell receptor in the immunogenic response to the peptide comprising that antigen (*i.e.*, the immunogen).

As used herein, "altered B-cell epitope," refers to an epitope amino acid sequence which differs from the precursor peptide or peptide of interest, such that the variant peptide of interest produces different (*i.e.*, altered) immunogenic responses in a human or another animal. It is contemplated that an altered immunogenic response includes altered immunogenicity and/or allergenicity (*i.e.*, an either increased or decreased overall immunogenic response). In some embodiments, the altered B-cell epitope comprises substitution and/or deletion of an amino acid selected from those residues within the identified epitope. In alternative embodiments, the altered B-cell epitope comprises an addition of one or more residues within the epitope.

As used herein "T-cell epitope" means a feature of a peptide or protein that is recognized by a T-cell receptor in the initiation of an immunologic response to the peptide comprising that antigen. Recognition of a T-cell epitope by a T-cell is generally believed to be via a mechanism wherein T-cells recognize peptide fragments of antigens which are bound to class I or class II Major Histocompatibility Complex (MHC) molecules expressed on antigen-presenting cells (*See e.g.*, Moeller (ed.), Immunol. Rev., 98:187 [1987]). In some embodiments of the present invention, the epitopes or epitopic fragments identified as described herein find use in the detection of antigen presenting cells having MHC molecules capable of binding and displaying the epitopes or fragments. In some embodiments, the epitopes/epitopic fragments further comprise a detectable label (*i.e.*, a marker) that facilitates the identification of cells that bind and/or display the epitope/epitopic fragment of interest.

As used herein, "T-cell proliferation," refers to the number of T-cells produced during the incubation of T-cells with the antigen presenting cells, with or without antigen.

"Baseline T-cell proliferation," as used herein, refers to the degree of T-cell proliferation that is normally seen in an individual in response to exposure to antigen presenting cells in the absence of peptide or protein antigen. For the purposes herein, the baseline T-cell proliferation level is determined on a per sample basis for each individual as the proliferation of T-cells in response to antigen presenting cells in the absence of antigen.

As used herein "altered immunogenic response," refers to an increased or reduced immunogenic response. Proteins and peptides exhibit an "increased immunogenic response" when the T-cell and/or B-cell response they evoke is greater than that evoked by a parental (*e.g.*, precursor) protein or peptide (*e.g.*, the protein of interest). The net result of this higher response is an increased antibody response directed against the variant protein or peptide. Proteins and peptides exhibit a "reduced immunogenic response" when the T-cell and/or B-cell response they evoke is less than that evoked by a parental (*e.g.*, precursor) protein or peptide. In preferred embodiments, the net result of this lower response is a reduced antibody response directed against the variant protein or peptide. In some preferred embodiments, the parental protein is a wild-type protein or peptide.

As used herein, an "*in vivo* reduction in immunogenicity" refers to an exhibited decrease in the immunogenic response as determined by an assay that occurs at least in part, within a living organism, (*e.g.*, requires the use of an living animal). Exemplary "*in vivo*" assays include determination of altered immunogenic responses in mouse models.

As used herein, an "*in vitro* reduction in immunogenicity" refers an exhibited decrease in the immunogenic response as determined by an assay that occurs in an artificial environment outside of a living organism (*i.e.*, does not require use of a living animal). Exemplary *in vitro* assays include testing the proliferative responses by human peripheral blood mononuclear cells to a peptide of interest.

As used herein, the term "significant epitope" refers to an epitope (*i.e.*, a T-cell and/or B-cell epitope) wherein the response rate within the tested donor pool is equal to or greater than about three times the background response rate.

As used herein, a "weakly significant epitope" refers to an epitope (*i.e.*, a T-cell and/or B-cell epitope), wherein the response rate within the tested donor pool is greater than the background response rate, but less than about three times the background rate.

As used herein, "protein of interest," refers to a protein which is being analyzed, identified and/or modified. Naturally-occurring, as well as recombinant proteins find use in the present invention.

As used herein, "protein" refers to any composition comprised of amino acids and recognized as a protein by those of skill in the art. The terms "protein," "peptide" and polypeptide are used interchangeably herein. Wherein a peptide is a portion of a protein, those skill in the art understand the use of the term in context. The term "protein" encompasses mature forms of proteins, as well as the pro- and prepro-forms of related proteins. Prepro forms of proteins comprise the mature form of the protein having a prosequence operably linked to the amino terminus of the protein, and a "pre-" or "signal" sequence operably linked to the amino terminus of the prosequence.

The variants of the present invention include the mature forms of protein variants, as well as the pro- and prepro- forms of such protein variants. The prepro- forms are the preferred construction since this facilitates the expression, secretion and maturation of the protein variants.

As used herein, "prosequence" refers to a sequence of amino acids bound to the N-terminal portion of the mature form of a protein which when removed results in the appearance of the "mature" form of the protein. Many enzymes are found in nature as translational proenzyme products and, in the absence of post-translational processing, are expressed in this fashion.

As used herein, "signal sequence" and "presequence" refer to any sequence of amino acids bound to the N-terminal portion of a protein or to the N-terminal portion of a pro-protein which may participate in the secretion of the mature or pro forms of the protein. This definition of signal sequence is a functional one and is intended to include all those amino acid sequences encoded by the N-terminal portion of the protein gene that participate in the effectuation of the secretion of protein under native conditions. The present invention utilizes such sequences to effect the secretion of the protein variants described herein.

As used herein, a "prepro" form of a protein variant consists of the mature form of the protein having a prosequence operably linked to the amino terminus of the protein and a "pre" or "signal" sequence operably linked to the amino terminus of the prosequence.

As used herein, functionally similar proteins are considered to be "related proteins." In some embodiments, these proteins are derived from a different genus and/or species, including differences between classes of organisms (*e.g.*, a bacterial protein and a fungal protein). In some embodiments, these proteins are derived from a different genus and/or species, including differences between classes of organisms (*e.g.*, a bacterial beta-lactamase and a fungal beta-lactamase). In additional embodiments, related proteins are provided from the same species. Indeed, it is not intended that the present invention be limited to related proteins from any particular source(s).

As used herein, the term "derivative" refers to a protein which is derived from a precursor protein by addition of one or more amino acids to either or both the C- and N-terminal end(s), substitution of one or more amino acids at one or a number of different sites in the amino acid sequence, and/or deletion of one or more amino acids at either or both ends of the protein or at one or more sites in the amino acid sequence, and/or insertion of one or more amino acids at one or more sites in the amino acid sequence. The preparation of a protein derivative is preferably achieved by modifying a DNA sequence which encodes for the native protein, transformation of that DNA sequence into a suitable host, and expression of the modified DNA sequence to form the derivative protein.

Related (and derivative) proteins comprise "variant proteins." In preferred embodiments, variant proteins differ from a parent protein and one another by a small number of amino acid residues. The number of differing amino acid residues may be one or more, preferably 1, 2, 3, 4, 5, 10, 15, 20, 30, 40, 50, or more amino acid residues. In one preferred embodiment, the number of different amino acids between variants is between 1 and 10. In particularly preferred embodiments, related proteins and particularly variant proteins comprise at least 80%, 85%, 90%, 95%, 97%, 98%, or 99% amino acid sequence identity. Additionally, a related protein or a variant protein as used herein, refers to a protein that differs from another related protein or a parent protein in the number of prominent regions. For example, in some embodiments, variant proteins have 1, 2, 3, 4, 5, or 10 corresponding prominent regions that differ from the parent protein.

In one embodiment, the prominent corresponding region of a variant produces only a background level of immunogenic response. Some of the residues identified for substitution, insertion or deletion are conserved residues whereas others are not. In the case of residues which are not conserved, the replacement of one or more amino acids is limited to substitutions which produce a variant which has an amino acid sequence that does not correspond to one found in nature. In the case of conserved residues, such replacements should not result in a naturally-occurring sequence.

In some embodiments, modification is preferably made to the "precursor DNA sequence" which encodes the amino acid sequence of the precursor enzyme, but can be by the manipulation of the precursor protein. In the case of residues which are not conserved, the replacement of one or more amino acids is limited to substitutions which produce a variant which has an amino acid sequence that does not correspond to one found in nature. In the case of conserved residues, such replacements should not result in a naturally-occurring sequence. Derivatives provided by the present invention further include chemical modification(s) that change the characteristics of the beta-lactamase.

In some embodiments, characteristics of variant beta-lactamases are determined by methods known to those skilled in the art. Exemplary characteristics include, but are not limited to thermal stability, alkaline stability, and stability of the particular beta-lactamase, various substrate, buffer solutions and/or product formulations. In combination with enzyme stability assays, variant beta-lactamases obtained through random mutagenesis can be identified which demonstrate either increased or decreased alkaline or thermal stability while maintaining enzymatic activity.

Alkaline stability can be measured either by known procedures or by the methods described herein. A substantial change in alkaline stability is evidenced by at least about a 5% or greater increase or decrease (in most embodiments, it is preferably an increase) in the half-life of the enzymatic activity of a mutant when compared to the precursor protein.

Thermal stability can be measured either by known procedures or by the methods described herein. A substantial change in thermal stability is evidenced by at least about a 5% or greater increase or decrease (in most embodiments, it is preferably an increase) in the half-life of the catalytic activity of a mutant when exposed to a relatively high temperature and neutral pH as compared to the precursor protein.

In some preferred embodiments, the beta-lactamase gene is ligated into an appropriate expression plasmid. The cloned beta-lactamase gene is then used to transform or transfect a host cell in order to express the beta-lactamase gene. This plasmid may replicate in hosts in the sense that it contains the well-known elements necessary for plasmid replication or the plasmid may be designed to integrate into the host chromosome. The necessary elements are provided for efficient gene expression (*e.g.*, a promoter operably linked to the gene of interest). In some embodiments, these necessary elements are supplied as the gene's own homologous promoter if it is recognized, (*i.e.*, transcribed, by the host), a transcription terminator (a polyadenylation region for eukaryotic host cells) which is exogenous or is supplied by the endogenous terminator region of the beta-lactamase gene. In some embodiments, a selection gene such as an antibiotic resistance gene that enables continuous cultural maintenance of plasmid-infected host cells by growth in antimicrobial-containing media is also included.

The following cassette mutagenesis method may be used to facilitate the construction of the beta-lactamase variants of the present invention, although other methods may be used. First, the naturally-occurring gene encoding the beta-lactamase is obtained and sequenced in whole or in part. Then, the sequence is scanned for a point at which it is desired to make a mutation (deletion, insertion or substitution) of one or more amino acids in the encoded beta-lactamase. The sequences flanking this point are evaluated for the presence of restriction sites for replacing a short segment of the gene with an oligonucleotide pool which when expressed will encode various mutants. Such restriction sites are preferably unique sites within the protein gene so as to facilitate the replacement of the gene segment. However, any convenient restriction site which is not overly redundant in the beta-lactamase gene may be used, provided the gene fragments generated by restriction digestion can be reassembled in proper sequence. If restriction sites are not present at locations within a convenient distance from the selected point (from 10 to 15 nucleotides), such sites are generated by substituting nucleotides in the gene in such a fashion that neither the reading frame nor the amino acids encoded are changed in the final construction. Mutation of the gene in order to change its sequence to conform to the desired sequence is accomplished by M13 primer extension in accord with generally known methods. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region which does not contain a site.

Once the naturally-occurring DNA or synthetic DNA is cloned, the restriction sites flanking the positions to be mutated are digested with the cognate restriction enzymes and a plurality of end termini-complementary oligonucleotide cassettes are ligated into the gene. The mutagenesis is simplified by this method because all of the oligonucleotides can be synthesized so as to have the same restriction sites, and no synthetic linkers are necessary to create the restriction sites.

As used herein, "corresponding to," refers to a residue at the enumerated position in a protein or peptide, or a residue that is analogous, homologous, or equivalent to an enumerated residue in a protein or peptide.

As used herein, "corresponding region," generally refers to an analogous position along related proteins or a parent protein.

The terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

As used herein, the term "analogous sequence" refers to a sequence within a protein that provides similar function, tertiary structure, and/or conserved residues as the protein of interest (*i.e.*, typically the original protein of interest). In particularly preferred embodiments, the analogous sequence involves sequence(s) at or near an epitope. For example, in epitope regions that contain an alpha helix or a beta sheet structure, the replacement amino acids in the analogous sequence preferably maintain the same specific structure. The term also refers to nucleotide sequences, as well as amino acid sequences. In some embodiments, analogous sequences are developed such that the replacement amino acids show a similar function, the tertiary structure and/or conserved residues to the amino acids in the protein of interest at or near the epitope. Thus, where the epitope region contains, for example, an alpha-helix or a beta-sheet structure, the replacement amino acids preferably maintain that specific structure.

As used herein, "homologous protein" refers to a protein (*e.g.*, beta-lactamase) that has similar action, structure, antigenic, and/or immunogenic response as a protein of interest (*e.g.*, an beta-lactamase from another source). It is not intended that homologs be necessarily related evolutionarily. Thus, it is intended that the term encompass the same functional protein obtained from different species. In some preferred embodiments, it is desirable to identify a homolog that has a tertiary and/or primary structure similar to the protein of interest, as replacement for the epitope in the protein of interest with an analogous segment from the homolog will reduce the disruptiveness of the change. Thus, in most cases, closely homologous proteins provide the most desirable sources of epitope substitutions. Alternatively, it is advantageous to look to human analogs for a given protein. For example, in some embodiments, substituting a specific epitope in one beta-lactamase with a sequence from another beta-lactamase or other species' beta-lactamase results in the production of beta-lactamase with reduced immunogenicity. In some preferred embodiments, the beta-lactamase homologs of the present invention have tertiary and/or primary structures substantially similar to wild-type beta-lactamase. A significant beta-lactamase epitope may be replaced with an analogous segment from a homologous enzyme. This type of replacement may reduce the disruptiveness of the change in the parent beta-lactamase. In most cases, closely homologous proteins provide the most desirable source of epitope substitutions.

As used herein, "homologous genes" refers to at least a pair of genes from different, but usually related species, which correspond to each other and which are identical or very similar to each other. The term encompasses genes that are separated by speciation (*i.e.*, the development of new species) (*e.g.*, orthologous genes), as well as genes that have been separated by genetic duplication (*e.g.*, paralogous genes). These genes encode "homologous proteins."

As used herein, "ortholog" and "orthologous genes" refer to genes in different species that have evolved from a common ancestral gene (*i.e.*, a homologous gene) by speciation. Typically, orthologs retain the same function in during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

The degree of homology between sequences may be determined using any suitable method known in the art (*See e.g.*, Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).

For example, PILEUP is a useful program to determine sequence homology levels. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle, (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps. Another example of a useful algorithm is the BLAST algorithm, described by Altschul *et al.*, (Altschul et al., J. Mol. Biol., 215:403-410, [1990]; and Karlin et al., Proc. Natl. Acad. Sci. USA 90:5873-5787 [1993]). One particularly useful BLAST program is the WU-BLAST-2 program (*See*, Altschul et al., Meth. Enzymol.,, 266:460-480 [1996]). parameters "W," "T," and "X" determine the sensitivity and speed of the alignment. The BLAST program uses as defaults a wordlength (W) of 11, the BLOSUM62 scoring matrix (*See,* Henikoff and Henikoff, Proc. Natl. Acad. Sci. USA 89:10915 [1989]) alignments (B) of 50, expectation (E) of 10, M'5, N'-4, and a comparison of both strands.

As used herein, "percent (%) nucleic acid sequence identity" is defined as the percentage of nucleotide residues in a candidate sequence that are identical with the nucleotide residues of the sequence.

As used herein, the term "hybridization" refers to the process by which a strand of nucleic acid joins with a complementary strand through base pairing, as known in the art.

As used herein, "hybridization conditions" refers to the conditions under which hybridization reactions are conducted. These conditions are typically classified by degree of "stringency" of the conditions under which hybridization is measured. The degree of stringency can be based, for example, on the melting temperature (Tm) of the nucleic acid binding complex or probe. For example, "maximum stringency" typically occurs at about Tm-5°C (5° below the Tm of the probe); "high stringency" at about 5-10° below the Tm; "intermediate stringency" at about 10-20° below the Tm of the probe; and "low stringency" at about 20-25° below the Tm. Alternatively, or in addition, hybridization conditions can be based upon the salt or ionic strength conditions of hybridization and/or one or more stringency washes. For example, 6xSSC = very low stringency; 3xSSC = low to medium stringency; 1xSSC = medium stringency; and 0.5xSSC = high stringency. Functionally, maximum stringency conditions may be used to identify nucleic acid sequences having strict identity or near-strict identity with the hybridization probe; while high stringency conditions are used to identify nucleic acid sequences having about 80% or more sequence identity with the probe.

For applications requiring high selectivity, one will typically desire to employ relatively stringent conditions to form the hybrids (*e.g.*, relatively low salt and/or high temperature conditions are used).

The phrases "substantially similar and "substantially identical" in the context of at least two nucleic acids or polypeptides typically means that a polynucleotide or polypeptide comprises a sequence that has at least 60% identity, preferably at least 75% sequence identity, more preferably at least 80%, yet more preferably at least 90%, still more preferably 95%, most preferably 97%, sometimes as much as 98% and 99% sequence identity, compared to the reference (*i.e.*, wild-type) sequence. Sequence identity may be determined using known programs such as BLAST, ALIGN, and CLUSTAL using standard parameters. (*See e.g.*, Altschul, et al., J. Mol. Biol. 215:403-410 [1990]; Henikoff et al., Proc. Natl. Acad Sci. USA 89:10915 [1989]; Karin et al., Proc. Natl Acad. Sci USA 90:5873 [1993]; and Higgins et al., Gene 73:237 - 244 [1988]). Software for performing BLAST analyses is publicly available through the National Center for Biotechnology Information. Also, databases may be searched using FASTA (Pearson et al., Proc. Natl. Acad. Sci. USA 85:2444-2448 [1988]). One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (*e.g.*, within a range of medium to high stringency).

As used herein, "equivalent residues" refers to proteins that share particular amino acid residues. For example, equivalent resides may be identified by determining homology at the level of tertiary structure for a protein (*e.g.*, IFN-β) whose tertiary structure has been determined by x-ray crystallography. Equivalent residues are defined as those for which the atomic coordinates of two or more of the main chain atoms of a particular amino acid residue of the protein having putative equivalent residues and the protein of interest (N on N, CA on CA, C on C and O on O) are within 0.13 nm and preferably 0.1 nm after alignment. Alignment is achieved after the best model has been oriented and positioned to give the maximum overlap of atomic coordinates of non-hydrogen protein atoms of the proteins analyzed. The preferred model is the crystallographic model giving the lowest R factor for experimental diffraction data at the highest resolution available, determined using methods known to those skilled in the art of crystallography and protein characterization/ analysis.

The present invention encompasses beta-lactamases having altered immunogenicity that are equivalent to those that are derived from the particular microbial strain mentioned. Being "equivalent," in this context, means that the beta-lactamases are encoded by a polynucleotide capable of hybridizing to the polynucleotide encoding the amino acid sequence of SEQ ID NO:1 under conditions of medium to high stringency and still retaining the altered immunogenic response to human T-cells. Thus, in some embodiments, equivalent beta-lactamases comprise at least 80%, at least 85%, at least 90%, at least 95%, at least 97% or at least 99% identity to the epitope sequences and the variant beta-lactamases having such epitopes.

As used herein, the terms "hybrid beta-lactamases" and "fusion beta-lactamases " refer to proteins that are engineered from at least two different or "parental" proteins. These parental proteins may be homologs of one another. For example, a hybrid beta-lactamase or fusion protein may contain the N-terminus of a protein and the C-terminus of a homolog of the protein. The two terminal ends may be combined to correspond to the full-length active protein. The homologs may share substantial similarity but do not have identical T-cell epitopes. Therefore, in a beta-lactamase of interest having one or more T-cell epitopes in the C-terminus, the C-terminus may be replaced with the C-terminus of a homolog having a less potent T-cell epitope, or fewer or no T-cell epitopes in the C-terminus. Thus, the skilled artisan understands that by being able to identify T-cell epitopes among homologs, a variety of variants producing different immunogenic responses can be formed. Moreover, it is understood that internal portions, and more than one homolog can be used to produce the variants of the present invention.

The term "regulatory element" as used herein refers to a genetic element that controls some aspect of the expression of nucleic acid sequences. For example, a promoter is a regulatory element which facilitates the initiation of transcription of an operably linked coding region. Additional regulatory elements include splicing signals, polyadenylation signals and termination signals.

As used herein, "expression vector" refers to a DNA construct containing a DNA sequence that is operably linked to a suitable control sequence capable of effecting the expression of the DNA in a suitable host. Such control sequences include a promoter to effect transcription, an optional operator sequence to control such transcription, a sequence encoding suitable mRNA ribosome binding sites and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. In the present specification, "plasmid" and "vector" are sometimes used interchangeably as the plasmid is the most commonly used form of vector at present. However, the invention is intended to include such other forms of expression vectors that serve equivalent functions and which are, or become, known in the art.

As used herein, "host cells" are generally prokaryotic or eukaryotic hosts which preferably have been manipulated by the methods known in the art (*See e.g.*, U.S. Patent 4,760,025 (RE 34,606)) to render them incapable of secreting enzymatically active endoprotease. A preferred host cell for expressing protein is the *Bacillus* strain BG2036 which is deficient in enzymatically active neutral protein and alkaline protease (subtilisin). The construction of strain BG2036 is described in detail in US Patent 5,264,366. Other host cells for expressing protein include *Bacillus subtilis* I168 (also described in US Patent 4,760,025 (RE 34,606) and US Patent 5,264,366,), as well as any suitable *Bacillus* strain, including, but not limited to those within the species of *B. licheniformis, B. lentus*, and other *Bacillus* species, etc.

However, it is not intended that the present invention be limited to *Bacillus* species as host cells, as other organisms are known in the art as suitable host cells (*e.g., E. coli*, etc.). In some preferred embodiments, the host cell has been modified such that endogenous beta-lactamase is not produced by the host cell.

Host cells are transformed or transfected with vectors constructed using recombinant DNA techniques known in the art. Transformed host cells are capable of either replicating vectors encoding the protein variants or expressing the desired protein variant. In the case of vectors which encode the pre- or prepro-form of the protein variant, such variants, when expressed, are typically secreted from the host cell into the host cell medium.

The term "introduced" in the context of inserting a nucleic acid sequence into a cell, means transformation, transduction or transfection. Means of transformation include protoplast transformation, calcium chloride precipitation, electroporation, naked DNA and the like as known in the art. (*See,* Chang and Cohen, Mol. Gen. Genet., 168:111-115 [1979]; Smith et al., Appl. Env. Microbiol., 51:634 [1986]; and the review article by Ferrari et al., in Bacillus Harwood (ed.), Plenum Publishing Corporation, pp. 57-52 [1989]).

The term "promoter/enhancer" denotes a segment of DNA which contains sequences capable of providing both promoter and enhancer functions (for example, the long terminal repeats of retroviruses contain both promoter and enhancer functions). The enhancer/promoter may be "endogenous" or "exogenous" or "heterologous." An endogenous enhancer/promoter is one which is naturally linked with a given gene in the genome. An exogenous (heterologous) enhancer/promoter is one which is placed in juxtaposition to a gene by means of genetic manipulation (*i.e.*, molecular biological techniques).

The presence of "splicing signals" on an expression vector often results in higher levels of expression of the recombinant transcript. Splicing signals mediate the removal of introns from the primary RNA transcript and consist of a splice donor and acceptor site (Sambrook et al., Molecular Cloning: A Laboratory Manual, 2nd ed., Cold Spring Harbor Laboratory Press, New York [1989], pp. 16.7-16.8). A commonly used splice donor and acceptor site is the splice junction from the 16S RNA of SV40.

The term "stable transfection" or "stably transfected" refers to the introduction and integration of foreign DNA into the genome of the transfected cell. The term "stable transfectant" refers to a cell which has stably integrated foreign or exogenous DNA into the genomic DNA of the transfected cell.

The terms "selectable marker" or "selectable gene product" as used herein refer to the use of a gene which encodes an enzymatic activity that confers resistance to an antibiotic or drug upon the cell in which the selectable marker is expressed.

As used herein, the terms "amplification" and "gene amplification" refer to a process by which specific DNA sequences are disproportionately replicated such that the amplified gene becomes present in a higher copy number than was initially present in the genome. In some embodiments, selection of cells by growth in the presence of a drug (*e.g.*, an inhibitor of an inhibitable enzyme) results in the amplification of either the endogenous gene encoding the gene product required for growth in the presence of the drug or by amplification of exogenous (*i.e.*, input) sequences encoding this gene product, or both. Gene amplification occurs naturally during development in particular genes such as the amplification of ribosomal genes in amphibian oocytes. Gene amplification may be induced by treating cultured cells with drugs. An example of drug-induced amplification is the methotrexate-induced amplification of the endogenous *dhfr* gene in mammalian cells (Schmike et al., Science 202:1051 [1978]). Selection of cells by growth in the presence of a drug (*e.g.*, an inhibitor of an inhibitable enzyme) may result in the amplification of either the endogenous gene encoding the gene product required for growth in the presence of the drug or by amplification of exogenous (*i.e.*, input) sequences encoding this gene product, or both.

"Amplification" is a special case of nucleic acid replication involving template specificity. It is to be contrasted with non-specific template replication (*i.e.*, replication that is template-dependent but not dependent on a specific template). Template specificity is here distinguished from fidelity of replication (*i.e.*, synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "co-amplification" refers to the introduction into a single cell of an amplifiable marker in conjunction with other gene sequences (*i.e.*, comprising one or more non-selectable genes such as those contained within an expression vector) and the application of appropriate selective pressure such that the cell amplifies both the amplifiable marker and the other, non-selectable gene sequences: The amplifiable-marker may be physically linked to the other gene sequences or alternatively two separate pieces of DNA, one containing the amplifiable marker and the other containing the non-selectable marker, may be introduced into the same cell.

As used herein, the terms "amplifiable marker," "amplifiable gene," and "amplification vector" refer to a marker, gene or a vector encoding a gene which permits the amplification of that gene under appropriate growth conditions.

As used herein, the term "amplifiable nucleic acid" refers to nucleic acids which may be amplified by any amplification method. It is contemplated that "amplifiable nucleic acid" will usually comprise "sample template."

As used herein, the term "sample template" refers to nucleic acid originating from a sample which is analyzed for the presence of "target" (defined below). In contrast, "background template" is used in reference to nucleic acid other than sample template which may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

"Template specificity" is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (*See e.g.*, Kacian et al., Proc. Natl. Acad. Sci. USA 69:3038 [1972]). Other nucleic acids are not replicated by this amplification enzyme. Similarly, in the case of T7 RNA polymerase, this amplification enzyme has a stringent specificity for its own promoters (*See*, Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (*See*, Wu and Wallace, Genomics 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e.*, in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (*i.e.*, a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (*e.g.*, ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the methods of U.S. Patent Nos. 4,683,195 4,683,202, and 4,965,188, which include methods for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (*i.e.*, denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified".

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

With PCR, it is possible to amplify a single copy of a specific target sequence in genomic DNA to a level detectable by several different methodologies (*e.g.*, hybridization with a labeled probe; incorporation of biotinylated primers followed by avidin-enzyme conjugate detection; incorporation of $^{32}$P-labeled deoxynucleotide triphosphates,

such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the terms "restriction endonucleases" and "restriction enzymes" refer to bacterial enzymes, each of which cut double-stranded DNA at or near a specific nucleotide sequence.

As used herein, "the genus *Bacillus*" includes all members known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis*. It is recognized that the genus *Bacillus* continues to undergo taxonomical reorganization. Thus, it is intended that the genus include species that have been reclassified, including but not limited to such organisms as *B. stearothermophilus*, which is now named "*Geobacillus stearothermophilus*." The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus*, and *Virgibacillus.*

As used herein, "effective amount of beta-lactamase enzyme" refers to the quantity of beta-lactamase enzyme necessary to achieve the enzymatic activity required in the specific application. Such effective amounts are readily ascertained by one of ordinary skill in the art and are based on many factors, such as the particular enzyme variant used, the application involved, the specific composition of the overall composition, etc.

As used herein, "pharmaceutically-acceptable" means that drugs, medicaments and/or inert ingredients which the term describes are suitable for use in contact with the tissues of humans and other animals without undue toxicity, incompatibility, instability, irritation, allergic response, and the like, commensurate with a reasonable benefit/risk ratio.

As indicated above, the beta-lactamases of the present invention exhibit modified immunogenic responses (*e.g.*, antigenicity and/or immunogenicity) when compared to the native beta-lactamases encoded by their precursor DNAs. In some preferred embodiments, the proteins (*e.g.*, beta-lactamases) exhibit reduced allergenicity/immunogenicity. Those of skill in the art readily recognize that the uses of the beta-lactamases of this invention will be determined, in large part, on the immunological properties of the proteins.

The epitopes identified herein may be used to elicit an immune response (*e.g.*, where it is desired to raise antibodies against an beta-lactamase) including one or both of such epitopes. Such antibodies find use in screening for other beta-lactamases that include one or both of these regions, or regions highly homologous thereto. In addition, the beta-lactamases of the present invention find use as reagents in various assays, such as immunoassays utilizing isolated natural epitope, recombinant protein, or synthetic peptide representing specific epitopic regions to evaluate persons for sensitization to proteins including these or highly homologous regions.

The epitopic fragments herein may be used in the detection of antigen presenting cells having MHC molecules capable of binding and displaying such fragments. For example, the epitopic fragments can include a detectable label (*e.g.*, radiolabel). The labeled fragments are then incubated with cells of interest, and then cells which bind (or display) the labeled fragments are detected.

In additional embodiments, the related and/or variant beta-lactamases with reduced allergenicity/immunogenicity find use in other applications, including pharmaceutical applications, drug delivery applications, and other health care applications.

In addition, the related and/or variant proteins with reduced allergenicity/immunogenicity find use in other applications, including pharmaceutical applications, drug delivery applications, and other health care applications. Indeed, it is contemplated that the beta-lactamases of the present invention will find widespread use in numerous compositions and applications.

## DETAILED DESCRIPTION OF THE INVENTION

The present invention provides beta-lactamase CD4+ T-cell epitopes, as well as novel variants that exhibit reduced immunogenic responses, as compared to the parental beta-lactamase. The present invention further provides DNA molecules that encode novel beta-lactamase variants, and host cells comprising DNA encoding novel beta-lactamase variants, as well as methods for making beta-lactamases less immunogenic. In addition, the present invention provides various compositions that comprise these beta-lactamase variants that are less immunogenic than the wild-type beta-lactamases. In some specific embodiments, the present invention provides beta-lactamase variants with reduced immunogenicity that are identified and/or characterized using the methods of the present invention.

Following the general assessment of the beta-lactamase T-cell epitopes, four T-cell epitopes in the beta-lactamase were further analyzed as described using the methods of the present invention. The following epitopes were determined to be of interest are listed in Table 1, below.

**Table 1. Peptides of Interest in Beta-Lactamase**

| Peptide # | Sequence | SEQ ID NO: |
|---|---|---|
| 6 | ITPLMKAQSVPGMAV | 2 |
| 36 | MLDLATYTAGGLPLQ | 3 |
| 49 | GTTRLYANASIGLFG | 4 |
| 107 | TGGFGSYVAFIPEKQ | 5 |

In some embodiments, the present invention further includes identifying residues that decrease the immunogenicity of beta-lactamase. In some embodiments, at least one amino acid substitution is made at least one T-cell epitope in the wild-type or parental beta-lactamase sequence. In some preferred embodiments, multiple amino acids are changed in the parental beta-lactamase sequence to produce an beta-lactamase variant having reduced allergenicity/immunogenicity. In alternative preferred embodiments, amino acid deletions, insertions and/or substitutions are made in the parental beta-lactamase sequence to produce a variant beta-lactamase with reduced allergenicity/immunogenicity. The beta-lactamase is from *E. cloacae*. In some embodiments, this beta-lactamase is wild-type, while in other embodiments, it is a mutated variant, conjugated variant, or a hybrid variant having amino acid substitutions in the epitope of interest, which can cause sensitization in an individual or sampling of individuals.

An epitope may be identified by an assay which identifies epitopes and non-epitopes as follows: differentiated dendritic cells are combined with naive human CD4+ and/or CD8+ T-cells and with a peptide of interest. More specifically, a reduced immunogenic response peptide of interest is provided wherein a T-cell epitope is recognized comprising the steps of: (a) obtaining from a single blood source a solution of dendritic cells and a solution of naive CD4+ and/or CD8+ T-cells; (b) differentiating the dendritic cells; (c) combining the solution of differentiated dendritic cells and the naïve CD4+ and/or CD8+ T-cells with a peptide of interest; and (d) measuring the proliferation of T-cells in step (c).

A series of peptide oligomers which correspond to all or part of the beta-lactamase of interest may be prepared. For example, a peptide library is produced covering the relevant portion or all of the protein. As described in the Examples below, a set of 15-mer peptides offset by three amino acids was used to identify the epitopes of interest. By analyzing each of the peptides individually in the assay provided herein, the methods of the present invention facilitate the precise identification of epitopes recognized by T-cells. In the example above, the greater reaction of one specific peptide than its neighbors' facilitates identification of the epitope anchor region to within three amino acids. In some embodiments, after the locations of these epitopes are determined, one or more of the amino acids within each epitope are modified, until the peptide produces a different T-cell response from that of the original protein. Moreover, the present invention provides means to identify proteins that have a desired low T-cell epitope potency which may be used in their naturally occurring forms (*i.e.*, wild-type proteins).

Those of skill in the art readily recognize the proteins and peptides of this invention are not necessarily native proteins and peptides. Indeed, in one embodiment of the present invention, shuffled genes having an altered immunologic response are contemplated (*See e.g.*, Stemmer, Proc. Natl. Acad. Sci. USA 91:10747 [1994]; Patten et al., Curr. Op. Biotechnol. 8:724 [1997]; Kuchner and Arnold, Trends Biotechnol., 15:523 [1997]; Moore et al., J. Mol. Biol., 272:336 [1997]; Zhao et al., Nature Biotechnol., 16:258 [1998]; Giver et al., Proc. Nat'l Acad. Sci. USA 95:12809 [1998]; Harayama, Trends BiotechnoL, 16:76 [1998]; Lin et al., Biotechnol., Prog., 15:467 [1999]; and Sun, Comput. Biol., 6:77 [1999], for descriptions of gene shuffling and expression of such genes). In some embodiments, beta-lactamases are altered such that the immune response generated by an animal against the beta-lactamase is altered.

Preferably, beta-lactamases according to the present invention are isolated or purified. By "purification" (or "isolation") it is meant that the beta-lactamase is altered from its natural state by virtue of separating the beta-lactamase from some or all of the naturally occurring constituents with which it is associated in nature. It is intended that this purification be accomplished by any suitable means known in the art, including but not limited to art-recognized separation techniques such as ion exchange chromatography, affinity chromatography, hydrophobic separation, dialysis, beta-lactamase treatment, ammonium sulfate precipitation or other protein salt precipitation, centrifugation, size exclusion chromatography, filtration, microfiltration, gel electrophoresis or separation on a gradient to remove whole cells, cell debris, impurities, extraneous proteins, and/or enzymes undesired in the final composition. In some embodiments, constituents are added to the beta-lactamase-containing composition to provide additional benefits, for example, activating agents, anti-inhibition agents, desirable ions, compounds to control pH, and/or other enzymes (*e.g.*, cellulase). In further embodiments, recombinant beta-lactamase is purified.

In addition to the above proteins and peptides, the present invention encompasses variant beta-lactamases that exhibit a reduced immunogenic response, provided that such beta-lactamases are as defined in the claims. The net result of this higher response is an increase in the antibodies directed against the variant beta-lactamase. These antibodies find use in various settings, including, but not limited to methods to detect beta-lactamase, beta-lactamase variants, etc. Variant beta-lactamases exhibit decreased immunogenic response when the T-cell response they evoke is less than that evoked by a parental (*i.e.*, precursor) beta-lactamase. The net result of this lower response is a decrease in the antibodies directed against the variant beta-lactamase. These variant beta-lactamases find use in various settings, including but not limited to administration to human and/or other animals in conjunction with other proteins.

Indeed, *in vivo* results described in Example 8 indicate that attenuating a proliferative response to an immunodominant epitope in mice results in the loss of immunogenicity of the whole protein at both the T cell proliferation and antibody production level. The level of proliferation seen to the CD1.1 protein that suggested a new epitope response was not enough to induce antibody responses in this immunization protocol. The epitope targeted was identical to an immunodominant epitope identified in the human population.

Exemplary assays useful in ascertaining the reduced immunogenic response of the variant proteins include, but are not limited to *in vivo* assays (HLA-DR3/DQ2 mouse T cell responses, and *in vitro* assays (human peripheral blood mononuclear cells (PBMC) to a sample of beta-lactamase and/or beta-lactamase variants. *In vivo* assays useful in ascertaining the reduced immunogenic response include, but are not limited to the use of transgenic mice, for example, rats (Taurog et al., Immunol. Rev., 169:209-223 [1999]), rabbits, or pigs. A preferred transgenic mouse model for testing modified proteins of interest and variants *in vivo,* determining a reduced immunogenic response, is the HLA-DR3/DQ2 mouse model known in the art.

In addition to the altering (*e.g.*, increasing or decreasing) the immunogenic response of an animal, such as a human, to naturally occurring amino acid sequences, the present invention provides means for reducing the immunogenic response of mutated proteins (*e.g.*, an beta-lactamase that has been altered to change the functional activity of the protein). In some embodiments, the mutation is made in order to provide some beneficial characteristic, including but not limited to increased activity, increased thermal stability, increased alkaline stability and/or oxidative stability. In some embodiments, the mutation results in the incorporation of new T-cell epitope in the mutated beta-lactamase. Dislosed herein are beta-lactamase T-cell epitopes and variant beta-lactamases with amino acid alterations that will alter the immunogenic response of the mutated protein.

Although this invention encompasses variant beta-lactamases as defined in the claims, for the sake of simplicity, the following will describe particularly preferred embodiments of the invention, which involve the modification of beta-lactamase.

The amino acid position numbers used herein refer to those assigned to the beta-lactamase sequence of SEQ ID NO:1. The invention, however, is not limited to the mutation of this particular beta-lactamase but extends to precursor beta-lactamases containing amino acid residues at positions that are "equivalent" to the particular identified residues in the beta-lactamase of SEQ ID NO:1. A residue (amino acid) of a precursor beta-lactamase is equivalent to a residue of a parental betalactamase if it corresponds in position in either primary or tertiary structure to a specific residue in the parental beta-lactamase. "Corresponding," as used herein generally refers to an analogous position along the peptide.

In some preferred embodiments, in order to establish homology to primary structure, the amino acid sequence of a precursor beta-lactamase is directly compared to the parental beta-lactamase primary sequence and particularly to a set of residues known to be invariant in beta-lactamases for which the sequence is known. After aligning the conserved residues, allowing for necessary insertions and deletions in order to maintain alignment (*i.e.*, avoiding the elimination of conserved residues through arbitrary deletion and insertion), the residues equivalent to particular amino acids in the primary sequence of the parental beta-lactamase are defined. Alignment of conserved residues preferably should conserve 100% of such residues. However, alignment of greater than 75% or as little as 50% of conserved residues is also adequate to define equivalent residues. These conserved residues, thus, may be used to define the corresponding equivalent amino acid residues of parental beta-lactamase in other beta-lactamases which are highly homologous to the preferred parental beta-lactamase.

In some embodiments, the present invention provides variant beta-lactamases having altered immunogenic response potential as compared to the precursor beta-lactamase(s). While the instant invention is useful to lower the immunogenic response, the mutations specified herein find use in combination with mutations known in the art to result altered thermal stability and/or altered substrate specificity, modified activity, improved specific activity or altered alkaline stability as compared to the precursor.

Two homologous proteins may be fused so as to eliminate at least one T-cell epitope. As is described below, a region of a protein in which a T-cell epitope resides may be replaced with the same region in a homologous protein that does not have the T-cell epitope. For example, a fusion protein is created with the parental beta-lactamase and a homolog, so that the resulting protein does not have the T-cell epitope present in the parental beta-lactamase. Any amino acid length can be used for fusion into the parental protein, from only the epitope to the majority of the protein, as long as the desired activity is maintained. However, it is not necessary that the original level of activity be maintained. Because of the lowered allergenicity/immunogenicity of the protein, in some embodiments, more of the hybrid protein is used than the parental protein, in order to achieve the same activity levels.

In some embodiments, the variant beta-lactamase activity is determined and compared with the beta-lactamase of interest by examining the interaction of the beta-lactamase with various commercial substrates, including, but not limited to casein, keratin, elastin, collagen. As desired, beta-lactamase activity can be determined by any suitable method known to those in the art. In additional embodiments, the other characteristics of the variant beta-lactamases are determined using suitable methods known to those in the art. Exemplary characteristics include, but are not limited to thermal stability, alkaline stability, and stability of the particular beta-lactamase in various substrate or buffer solutions or product formulations. When combined with the enzyme stability assay procedure disclosed herein, mutants obtained by random mutagenesis are identified which demonstrated either increased or decreased alkaline or thermal stability while maintaining enzymatic activity.

In some embodiments, the present invention provides beta-lactamase compositions with reduced immunogenicity, further comprising recognition sites for antibodies and/or cell receptors. In some preferred embodiments, the reduced immunogenicity beta-lactamase is conjugated to at least a portion of an antibody. This embodiment finds particular use in the targeted recognition of cells. In some embodiments, the cells recognized by the antibody are abnormal cells, including but not limited to malignant cells. Upon recognition of the reduced immunogenicity beta-lactamase, the enzyme converts a non-toxic prodrug into a cytotoxic component (*e.g.*, melphalan). This results in the destruction of the cell by the cytotoxic component. In some embodiments, the beta-lactamase is modified so as to contain at least one cancer-targeting sequence. In this embodiment, the patient (*e.g.*, cancer patient) is administered a beta-lactamase sequence that has been modified so as to directly recognize abnormal (*e.g.*, cancer) cells. Thus, the present invention provides means to directly target cells for destruction and removal.

**EXPERIMENTAL**

The following examples serve to illustrate certain preferred embodiments and aspects of the present invention and are not to be construed as limiting the scope thereof.

In the experimental disclosure which follows, the following abbreviations apply: sd and SD (standard deviation); M (molar); mM (millimolar); $\mu$M (micromolar); nM (nanomolar); mol (moles); mmol (millimoles); $\mu$mol (micromoles); nmol (nanomoles); gm (grams); mg (milligrams); $\mu$g (micrograms); pg (picograms); L (liters); ml (milliliters); $\mu$l (microliters); cm (centimeters); mm (millimeters); $\mu$m (micrometers); nm (nanometers); °C (degrees Centigrade); cDNA (copy or complimentary DNA); DNA (deoxyribonucleic acid); ssDNA (single stranded DNA); dsDNA (double stranded DNA); dNTP (deoxyribonucleotide triphosphate); RNA (ribonucleic acid); PBS (phosphate buffered saline); g (gravity); OD (optical density); CPM and cpm (counts per minute); rpm (revolutions per minute); Dulbecco's phosphate buffered solution (DPBS); HEPES (N-[2-Hydroxyethyl]piperazine-N-[2-ethanesulfonic acid]); HBS (HEPES buffered saline); SDS (sodium dodecylsulfate); Tris-HCl (tris[Hydroxymethyl]aminomethane-hydrochloride); 2-ME (2-mercaptoethanol); EGTA (ethylene glycol-bis($\beta$-aminoethyl ether) N, N, N', N'-tetraacetic acid); EDTA (ethylenediaminetetracetic acid); SI (stimulation index); bla ($\beta$-lactamase or ampicillin-resistance gene); PBMC (peripheral blood mononuclear cell); Endogen (Endogen, Woburn, MA); CytoVax (CytoVax, Edmonton, Canada); Wyeth-Ayerst (Wyeth-Ayerst, Philadelphia, PA); NEN (NEN Life Science Products, Boston, MA); Wallace Oy (Wallace Oy, Turku, Finland); Pharma AS (Pharma AS, Oslo, Norway); Dynal (Dynal, Oslo, Norway); Abbott (Abbott Laboratories, Abbott Park, IL); Bio-Synthesis (Bio-Synthesis, Lewisville, TX); ATCC (American Type Culture Collection, Rockville, MD); Gibco/BRL (Gibco/BRL, Grand Island , NY); Sigma (Sigma Chemical Co., St. Louis, MO); Pharmacia (Pharmacia Biotech, Piscataway, NJ); and Stratagene (Stratagene, La Jolla, CA).

Peptides

All peptides were obtained from a commercial source (Mimotopes, San Diego, CA). For the I-MUNE® assay system described herein, 15-mer peptides offset by 3 amino acids that described the entire sequence of the proteins of interest were synthesized in a multipin format *(See,* Maeji et al., J. Immunol. Meth., 134:23-33 [1990]). Peptides were resuspended in DMSO at approximately 1 to 2 mg/ml, and stored at -70°C prior to use. Each peptide was tested at least in duplicate. The results for each peptide were averaged. In some cases, the stimulation index (SI) was calculated for each peptide.

**Human Donor Blood Samples**

Volunteer donor human blood buffy coat samples were obtained from two commercial sources (Stanford Blood Center, Palo Alto, CA, and the Sacramento Medical Foundation, Sacramento, CA). Buffy coat samples were further purified by density separation. Each sample was HLA typed for HLA-DR and HLA-DQ using a commercial PCR-based kit (Bio-Synthesis). The HLA DR and DQ expression in the donor pool was determined to not be significantly different from a North American reference standard (Mori et al., Transplant., 64:1017-1027 [1997]). However, the donor pool did show evidence of slight enrichments for ethnicities common to the San Francisco Bay Area.

**Data Analysis**

For each individual buffy coat sample, the average CPM values for all of the peptides were analyzed. The average CPM values for each peptide were divided by the average CPM value for the control (DMSO only) wells to determine the "stimulation index" (SI). Donors were tested with each peptide set until an average of at least two responses per peptide were compiled. The data for each protein was graphed showing the percent responders to each peptide within the set. A positive response was collated if the SI value was equal to or greater than 2.95. This value was chosen as it approximates a difference of three standard deviations in a normal population distribution. For each protein assessed, positive responses to individual peptides by individual donors were compiled. To determine the background response for a given protein, the percent responders for each peptide in the set were averaged and a standard deviation was calculated. Statistical significance was calculated using Poisson statistics for the number of responders to each peptide within the dataset. Different statistical methods were used as described herein. The response to a peptide was considered significant if either the number of donors responding to the peptide was different from the Poisson distribution defined by the dataset with a $p < 0.05$ and/or if the percent response was at least 3-fold greater than the background.

**Statistical Methods**

Statistical significance of peptide responses were calculated based on Poisson statistics. The average frequency of responders was used to calculate a Poisson distribution based on the total number of responses and the number of peptides in the set. A response was considered significant if $p < 0.05$. In addition, two-tailed Student's t-tests with unequal variance, were performed. For epitope determination using data with low background response rates, a conservative Poisson based formula was applied: $= 1 - e\left(-n\left(1 - \sum \frac{\lambda^x e^{-\lambda}}{x!}\right)\right)$ where $n$ = the number of peptides in the set, $x$ =

the frequency of responses at the peptide of interest, and $\lambda$ = the median frequency of responses within the dataset. For epitope determinations based on data with a high background response rate, the less stringent Poisson based determination $1 - \left(\sum_{i=0}^{x} \frac{\lambda^x e^{-\lambda}}{x!}\right)$ was used, where $\lambda$ = the median frequency of responses in the dataset, and x = the frequency of responses at the peptide of interest.

In additional embodiments, the structure determination is calculated based on the following formula:

$$\sum \left| f(i) - \frac{1}{p} \right|$$

wherein $\Sigma$ (upper case sigma) is the sum of the absolute value of the frequency of responses to each peptide minus the frequency of that peptide in the set; $f(i)$ is defined as the frequency of responses for an individual peptide; and $p$ is the number of peptides in the peptide set.

This equation returns a value between 0 and 2, which is equal to the "Structure Value." A value of 0 indicates that the results are completely without structure, and a value of 2.0 indicates all structure is highly structured around a single area. The closer the value is to 2.0, the more immunogenic the protein. Thus, a low value indicates a less immunogenic protein.

**HLA Types Within the Donor Pool**

HLA-DR and DQ types were analyzed for associations with responses to defined epitope peptides. A Chi-squared analysis, with one degree of freedom was used to determine significance. Where an allele was present in both the responder and non-responder pools, a relative risk was calculated.

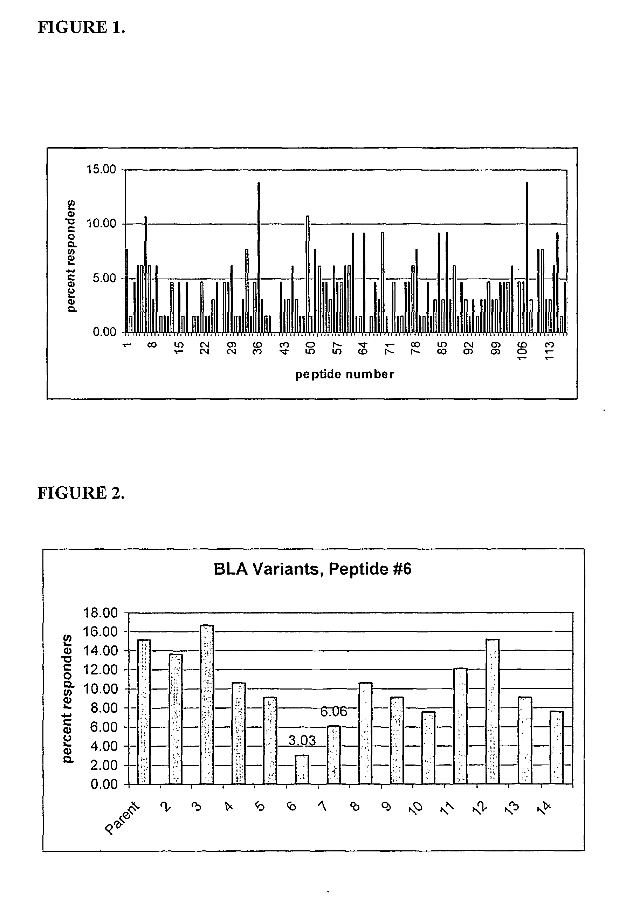

The HLA-DRB1 allelic expression was determined for approximately 185 random individuals. HLA typing was performed using low-stringency PCR determinations. PCR reactions were performed as directed by the manufacturer (Bio-Synthesis). The data compiled for the Stanford and Sacramento samples were compared the "Caucasian" HLA-DRB1 frequencies as published (*See*, Marsh et al., HLA Facts Book, The, Academic Press, San Diego, CA [2000], page 398, Figure 1). The donor population in these communities is enriched for HLA-DR4 and HLA-DR15. However, the frequencies of these alleles in these populations are well within the reported range for these two alleles (5.2 to 24.8% for HLA-DR4 and 5.7 to 25.6% for HLA-DR15). Similarly, for HLA-DR3, -DR7 and DR11, the frequencies are lower than the average Caucasian frequency, but within the reported ranges for those alleles. Also of note, HLA DR15 is found at a higher frequency in ethnic populations that are heavily represented in the San Francisco Bay Area.

**EXAMPLE 1**

**Preparation of Cells Used in the I-MUNE® Assay System for the Identification of Peptide T-Cell Epitopes in Beta-Lactamase Using Human T-Cells**

Fresh human peripheral blood cells were collected from 69 humans of unknown exposure status to beta-lactamase. These cells were tested to determine antigenic epitopes in beta-lactamase, as described in Example 3.

Peripheral mononuclear blood cells (stored at room temperature, no older than 24 hours) were prepared for use as follows. For each sample, approximately 30 mls of a solution of buffy coat preparation from one unit of whole blood was brought to 50 ml with Dulbecco's phosphate buffered solution (DPBS) and split into two tubes. The samples were underlaid with 12.5 ml of room temperature Lymphoprep density separation media (Nycomed; Pharma AS; Density 1.077 g/ml). The tubes were centrifuged for thirty minutes at 600 xg. The interface of the two phases was collected, pooled and washed in DPBS. The cell density of the resultant solution was measured by hemocytometer, as known in the art. Viability was measured by trypan blue exclusion, as known in the art.

From the resulting solution, a differentiated dendritic cell culture was prepared from the peripheral blood mononuclear cell sample having a density of $10^8$ cells per 75 ml culture flask in a solution as described below:

(1) 50 ml of serum free AIM V media (Gibco) was supplemented with a 1:1000 dilution beta-mercaptoethanol (Gibco). The flasks were laid flat for two hours at 37°C in 5% $CO_2$ to allow adherence of monocytes to the flask wall.

(2) Differentiation of the monocyte cells to dendritic cells was performed as follows: nonadherent cells were removed and the resultant adherent cells (monocytes) combined with 30 ml of AIM V, 800 units/ml of GM-CSF (Endogen) and 500 units/ml of IL-4 (Endogen); the resulting mixture was cultured for 5 days at 37°C in 5% $CO_2$. After the five days of incubation, the cytokine TNF$\alpha$ (Endogen) was added to 0.2 units/ml, and the cytokine IL-1$\alpha$ (Endogen) was added to a final concentration of 50 units/ml and the mixture incubated at 37°C in 5% $CO_2$ for two more days.

(3) On the seventh day, mitomycin C was added to a concentration of 50 micrograms/ml in 100 mM EDTA-containing PBS to stop growth of the now differentiated dendritic cell culture. The solution was incubated for 60 minutes at 37°C in 5% $CO_2$. Dendritic cells were dislodged from the plastic surface by gently rapping the flask. Dendritic cells were then centrifuged at 600 xg for 5 minutes, washed in DPBS and counted as described above.

(4) The prepared dendritic cells were placed into a 96 well round bottom plate at a concentration of $2x10^4$ cells/well in 100 microliter total volume of AIM V media, per well.

CD4+ T cells were prepared from frozen aliquots of the peripheral blood cell samples used to prepare the dendritic cells, using reagents provided by the Dynal CD4+ T-cell enrichment kit (Dynal). The resultant CD4+ cell solution was centrifuged, resuspended in AIMV media and the cell density was determined using methods known in the art. The CD4+ T-cell suspension was then resuspended to a count of $2x10^6$/ml in AIM V media to facilitate efficient manipulation of 96-well plates.

**EXAMPLE 2**

**Identification of T-Cell Epitopes in Beta-Lactamase**

Peptides for use in the I-MUNE® assay described in Example 3 were prepared based on the sequence of beta-lactamase precursor (cephalosporinase) obtained from *Enterobacter cloacae,* GenBank Accession No. P05364, with the sequence:

TPVSEKQLAE VVANTITPLM KAQSVPGMAV AVIYQGKPHY YTFGKADIAA NKPVTPQTLF
ELGSISKTFT GVLGGDAIAR GEISLDDAVT RYWPQLTGKQ WQGIRMLDLA TYTAGGLPLQ
VPDEVTDNAS LLRFYQNWQP QWKPGTTRLY ANASIGLFGA LAVKPSGMPY EQAMTTRVLK
PLKLDHTWIN VPKAEEAHYA WGYRDGKAVR VSPGMLDAQA YGVKTNVQDM ANWVMANMAP
ENVADASLKQ GIALAQSRYW RIGSMYQGLG WEMLNWPVEA NTVVEGSDSK VALAPLPVAE
VNPPAPPVKA SWVHKTGSTG GFGSYVAFIP EKQIGIVMLA NTSYPNPARV EAAYHILEAL Q
(SEQ ID NO:1).

Based upon the full length amino acid sequence (SEQ ID NO:1) of this beta-lactamase, a set of 15mers off-set by three amino acids comprising the entire sequence of beta-lactamase were synthetically prepared by Mimotopes.

Peptide antigen was prepared as a 2 mg/ml stock solution inDMSO. First, 0.5 microliters of the stock solution were placed in each well of the 96 well plate in which the differentiated dendritic cells were previously placed. Then, 100 microliters of the diluted CD4+ T-cell solution as prepared above, were added to each well. Useful controls include diluted DMSO blanks, and tetanus toxoid positive controls.

The final concentrations in each well, at 20 microliter total volume are as follows:

$2 \times 10^4$ CD4+
$2 \times 10^5$ dendritic cells (R:S of 10:1)
5 $\mu$M peptide

**EXAMPLE 3**

**1-MUNE® Assay for the Identification of Peptide T-Cell Epitopes in Beta-Lactamase Using Human T-Cells**

Once the assay reagents (*i.e.*, cells, peptides, etc.) were prepared and distributed into the 96-well plates, the I-MUNE® assays were conducted. Controls included dendritic cells plus CD4+ T-cells alone (with DMSO carrier) and with tetanus toxoid (Wyeth-Ayerst), at approximately 5 Lf/mL.

Cultures were incubated at 37°C in 5% $CO_2$ for 5 days. Tritiated thymidine (NEN) was added at 0.5 microCi/well. The cultures were harvested and assessed for incorporation the next day, using the Wallac TriBeta scintillation detection system (Wallace Oy).

All tests were performed at least in duplicate. All tests reported displayed robust positive control responses to the antigen tetanus toxoid. Responses were averaged within each experiment, then normalized to the baseline response. A positive event (*i.e.*, a proliferative response) was recorded if the response was at least 2.95 times the baseline response.

The immunogenic responses (*i.e.*, T-cell proliferation) to the prepared peptides from beta-lactamase were tallied and are shown in Figure 1. The overall background rate of responses to this peptide set was 4.04% for the donors tested. Using these methods various peptides of potential interest were identified, including those in Table 2, below.

**Table 2. Peptides of Interest in Beta-Lactamase**

| Peptide # | Sequence | SEQ ID NO: |
|---|---|---|
| 6 | ITPLMKAQSVPGMAV | 2 |
| 36 | MLDLATYTAGGLPLQ | 3 |
| 49 | GTTRLYANASIGLFG | 4 |
| 107 | TGGFGSYVAFIPEKQ | 5 |

Peptides #36 and #107 were determined to be significant (p<0.05), by both conservative ((1-EXP(-peptide number* (1-POISSON(value, mean, cumulative))) and non-conservative (1-POISSON(value mean, cumulative)) statistical methods (these are Excel® spreadsheet formulae). The responses to these peptides were both 3x above the background (the response was 12.11%), and background + 3 standard deviations (sd= 2.87%, 3 sd=12.62%). Peptides #6 and #49 both reached statistical significance using less conservative analyses (p<0.05 for both). The statistical analyses used are those described above.

As further described herein, it is contemplated that amino acid modifications in or around these peptides will provide variant beta-lactamases suitable for use as hypo-allergenic/immunogenic beta-lactamases.

**EXAMPLE 4**

**HLA Association with an Epitope Peptide Number**

The HLA-DR and DQ expression of 65 of the donors tested in both rounds of assay testing described above were assessed using a commercially available PCR-based HLA typing kit (Bio-Synthesis). The phenotypic frequencies of individual HLA-DRB1 and DQB1 antigens among responders and non-responders to four epitopes (peptides #6, #36, #49, and #107) were tested using a chi-squared analysis with 1 degree of freedom. Wherever the HLA antigen was present in both reactive and non-reactive donors, a relative risk (*i.e.*, the increased or decreased likelihood of presenting a reaction conditioned on the presence of the HLA antigen) was computed. Allele frequencies among donors that reacted and did not react to the specific epitopes were also computed. The effect of HLA antigens in the quantitative responses to peptides #6, #36, #49, and #107 were tested using a one-sided t-test. In addition, the mean and standard error of quantitative response for each peptide were determined.

In some embodiments, the phenotypic frequencies of individual HLA-DR and -DQ antigens among responders and non-responders to a peptide number are tested using a chi-squared analysis with 1 degree of freedom. The increased or decreased likelihood of reacting to an epitope corresponding to the peptide number is calculated wherever the HLA antigen in question is present in both responding and non-responding donor samples and the corresponding epitope is considered an HLA associated epitope.

The magnitude of the proliferative response to an individual peptide in responders and non-responders expressing epitope-associated HLA alleles were also be analyzed. An "individual responder to the peptide" is defined by a stimulation index of greater than 2.95. It is contemplated that the proliferative response in donors who express an epitope associated with HLA alleles are higher than in peptide responders who do not express the associated allele.

Statistically significant ($p<0.05$) correlations were observed between some DR and DQ antigens and peptides #107, and #49. Although there were some differences in antigen carrier frequencies between responders and non-responders to peptides #36 and #6, these did not reach statistical significance. The strongest association was found between reaction to peptide #107 and the presence of DR8, with 33% in the reaction group, compared to 2% in the non-reaction group ($p<0.0003$). The increased likelihood of a DR8+ individual relative to a DR8- individual to respond to this peptide was 7.63.

DR9 was increased among subjects reactive to epitope #49, with 28.6% in the reaction group and 3.4% in the non-reaction group ($p<0.009$). The relative risk was found to be 6.1.

DR1 was associated with responses to one or more peptides, although none were statistically significant (26% in the reaction group and 9% in the non-reaction group; $p<0.07$). DR1 was found to be increased among donors who responded to one or more of all four peptides (26% vs. 9%), although the difference did not reach statistical significance ($p<0.07$; with a relative risk of 1.71). As DR1 was found to be associated with a higher quantitative response among responders to peptides #36 and #107, it is contemplated that this epitope may be involved in the risk of allergy to beta-lactamase. Although not quite statistically significant, it is of interest that DR1 was associated with a 27% increased quantitative response among donors reactive to peptide #107 (5.4 compared to 4.2). For peptide #36, DR1+ responders had a 76% (7.8 compared to 4.42) higher response, relative to DR1- responders, although the presence of this allele has not been found to be significantly associated with response to this or any other peptide.

Among the non-responders to peptide #107, DR13 was found to be associated with a particularly low response, as it was found to be 23% lower than the other genotypes.

The presence of DR13, but absence of DQ6 (*i.e.*, DR13+ and DQ6-) was significantly associated with responses to at least two peptides (37% compared to 9%; $p<0.028$), which is statistically significant. The relative risk for this combination was found to be 3.98. For the combination of DR13+ and DQ6-, was increased among responders to at least one of the 5 peptides ($p<.14$). DR13 appears to have an important role in allergy to beta-lactamase, but only in haplotypes that do not carry DQ6.

Indeed, DQ6 was completely absent from among donors responding to peptide #107, yet was found in 37.5% of non-responders ($p<0.03$). The combination of DR13+ and DQ6- was increased, although not significantly among responders to peptide #49 (28% compared to 10%).

DQ4 was increased among individuals that reacted to peptide #36 (22% compared to 7%; $p<0.15$), but this difference did not reach statistical significance. For peptide #6, although no allele was significantly associated with this peptide, DR4 was increased among donors who responded to this peptide (57% reactive, compared to 26% non-reactive; $p<0.09$), with an associated relative risk of 3.5.

The presence of DR1 was found to correlate with a higher quantitative response (compared with other genotypes) among responsive donors to peptides #107 (27%) and #36 (36%). Although individually, DR1 was not associated with any specific allele, taken together, these findings indicate that DR1 may be important in defining the response to beta-lactamase.

From the above, it is clear that the present invention provides methods and compositions for the identification of T-cell epitopes in wild-type beta-lactamase. Once antigenic epitopes are identified, the epitopes are modified as desired, and the peptide sequences of the modified epitopes incorporated into a wild-type beta-lactamase, so that the modified sequence is no longer capable of initiating the CD4[+] T-cell response or wherein the CD4[+]T -cell response is significantly reduced in comparison to the wild-type parent. In particular, the present invention provides means, including methods and compositions suitable for reducing the immunogenicity of beta-lactamase.

## EXAMPLE 5

**Critical Residue Testing**

In this Example, critical residue testing experiments for variants of peptides #6, #36, #49, and #107. In these experiments, alanine scans were performed for each peptide in order to produce variants of each of the parent peptides (*i.e.*, peptides #6, #36, #40 and #107). These variant peptides were synthesized by Mimotopes (San Diego, CA) using the multi-pin synthesis technique known in the art (*See e.g.,* Maeji et al., J. Immunol. Meth., 134:23-33 [1990]).

The assay was performed as described in Example 3, utilizing the variant peptides on a set of 66 donor samples. Proliferative responses were collated, and the results described in greater detail below.

For peptide #6 (SEQ ID NO:2), the following sequences in Table 3 were tested. Of these, sequences #6 and #7 (SEQ ID NOS:10 and 11) were found to be of interest. The results of the assay with these peptide variants are shown in Figure 2.

### Table 3. Peptide #6 and Variants

| Sequence # | Sequence | SEQ ID NO: |
|---|---|---|
| parent | ITPLMKAQSVPGMAV | 2 |
| 2 | ATPLMKAQSVPGMAV | 6 |
| 3 | IAPLMKAQSVPGMAV | 7 |
| 4 | ITALMKAQSVPGMAV | 8 |
| 5 | ITPAMKAQSVPGMAV | 9 |
| 6 | ITPLAKAQSVPGMAV | 10 |
| 7 | ITPLMAAQSVPGMAV | 11 |
| 8 | ITPLMKAASVPGMAV | 12 |
| 9 | ITPLMKAQAVPGMAV | 13 |
| 10 | ITPLMKAQSAPGMAV | 14 |
| 11 | ITPLMKAQSVAGMAV | 15 |
| 12 | ITPLMKAQSVPAMAV | 16 |
| 13 | ITPLMKAQSVPGAAV | 17 |
| 14 | ITPLMKAQSVPGMAA | 18 |

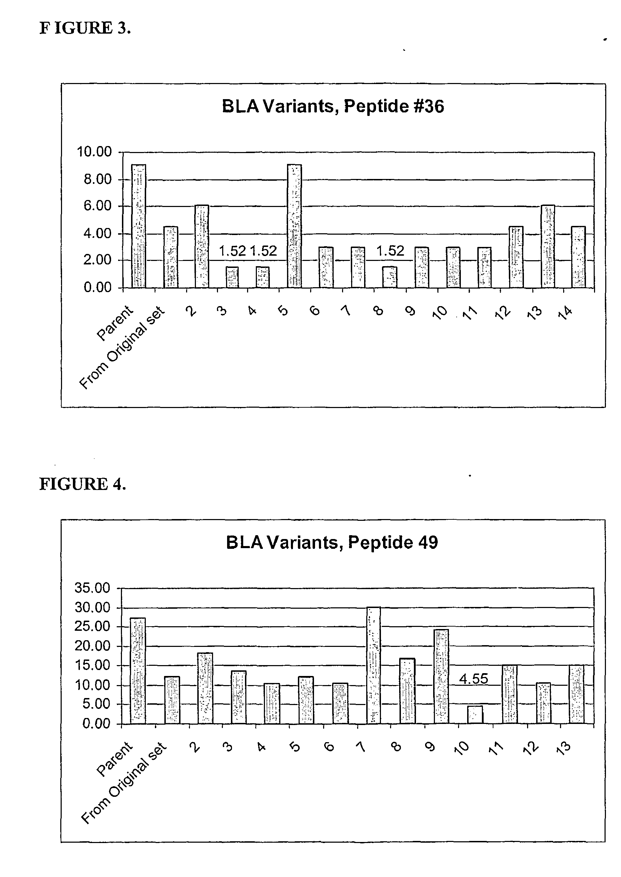

For peptide #36 (SEQ ID NO:3), the following sequences in Table 4 were tested. Of these, sequences #3, #4 and #8 (SEQ ID NOS:20, 21, and 25) were found to be of interest. The results of the assay with these peptide variants is shown in Figure 3.

### Table 4. Peptide #36 and Variants

| Sequence # | Sequence | SEQ ID NO: |
|---|---|---|
| parent | MLDLATYTAGGLPLQ | 3 |
| 2 | ALDLATYTAGGLPLQ | 19 |
| 3 | MADLATYTAGGLPLQ | 20 |
| 4 | MLALATYTAGGLPLQ | 21 |
| 5 | MLDAATYTAGGLPLQ | 22 |
| 6 | MLDLAAYTAGGLPLQ | 23 |
| 7 | MLDLATATAGGLPLQ | 24 |
| 8 | MLDLATYAAGGLPLQ | 25 |
| 9 | MLDLATYTAAGLPLQ | 26 |
| 10 | MLDLATYTAGALPLQ | 27 |
| 11 | MLDLATYTAGGAPLQ | 28 |
| 12 | MLDLATYTAGGLALQ | 29 |
| 13 | MLDLATYTAGGLPAQ | 30 |
| 14 | MLDLATYTAGGLPLA | 31 |

For peptide #49 (SEQ ID NO:4), the following sequences in Table 5 were tested. Of these, sequences, peptide 10 (SEQ ID NO:40) was found to be of interest. The results of the assay with these peptide variants is shown in Figure 4.

**Table 5. Peptide #49 and Variants**

| Sequence | Sequence | SE Q ID NO: |
|---|---|---|
| parent | GTTRLYANASIGLFG | 4 |
| 2 | ATTRLYANASIGLFG | 32 |
| 3 | GATRLYANASIGLFG | 33 |
| 4 | GTARLYANASIGLFG | 34 |
| 5 | GTTALYANASIGLFG | 35 |
| 6 | GTTRAYANASIGLFG | 36 |
| 7 | GTTRLAANASIGLFG | 37 |
| 8 | GTTRLYAAASIGLFG | 38 |
| 9 | GTTRLYANAAIGLFG | 39 |
| 10 | GTTRLYANASAGLFG | 40 |
| 11 | GTTRLYANASIGAFG | 41 |
| 12 | GTTRLYANASIGLAG | 42 |
| 13 | GTTRLYANASIGLFA | 43 |

For this epitope, as described in the following Example, specific amino acid substitutions were tested in the I-MUNE® assay (see above) on an additional set of 69 donors along with the alanine scan mutagenized peptides. These peptides were tested as 15 -mer peptides offset by 3 amino acids across the peptide sequence of beta-lactamase that encompasses epitope #49. These tests were performed in order to ensure that the amino acid variants did not introduce a *de novo* CD4+ T-cell epitope in another frame.

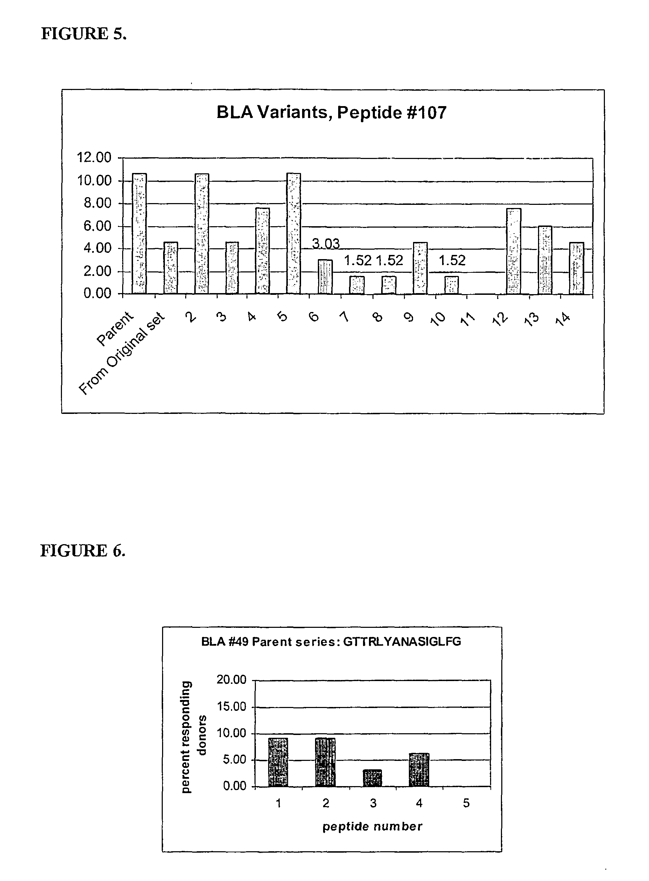

For peptide #107, the following sequences in Table 6 were tested. Of these, sequences 6, 7, 8, 10, and 11 (SEQ ID NOS: 48,49,50,52, and 53) were found to be of interest. The results of the assay with these peptide variants is shown in Figure 5.

**Table 6. Peptide #107 and Variants**

| Sequence # | Sequence | SEQ ID NO: |
|---|---|---|
| parent | TGGFGSYVAFIPEKQ | 5 |
| 2 | TAGFGSYVAFIPEKQ | 44 |
| 3 | TGAFGSYVAFIPEKQ | 45 |
| 4 | TGGAGSYVAFIPEKQ | 46 |
| 5 | TGGFASYVAFIPEKQ | 47 |
| 6 | TGGFGAYVAFIPEKQ | 48 |
| 7 | TGGFGSAVAFIPEKQ | 49 |
| 8 | TGGFGSYAAFIPEKQ | 50 |
| 9 | TGGFGSYVAAIPEKQ | 51 |
| 10 | TGGFGSYVAFAPEKQ | 52 |
| 11 | TGGFGSYVAFIAEKQ | 53 |
| 12 | TGGFGSYVAFIPAKQ | 54 |
| 13 | TGGFGSYVAFIPEAQ | 55 |
| 14 | TGGFGSYVAFIPEKA | 56 |

In view of the above information, the following peptides were selected as potential variant sequences to reduce the immunogenic potential of the beta-lactamase epitopes.

**Table 7. Variant Sequences with Potentially Reduced Immunogenicity**

| Epitope Peptide | Parent Sequence | Variant Sequence |
|---|---|---|
| #6 | ITPLMKAQSVPGMAV (SEQ ID NO:2) | ITPLAKAQSVPGMAV (SEQ ID NO:10) |
| | | ITPLMAAQSVPGMAV (SEQ ID NO:11) |

(continued)

| Epitope Peptide | Parent Sequence | Variant Sequence |
|---|---|---|
| #36 | MLDLATYTAGGLPLQ (SEQ ID NO:3) | MADLATYTAGGLPLQ (SEQ ID NO:20) |
| | | MLALATYTAGGLPLQ (SEQ ID NO:21) |
| | | MLDLATYAAGGLFLQ (SEQ ID NO:25) |
| #49 | GTTRLYANASIGLFG (SEQ ID NO:4) | GTTRLYANASFGLFG (SEQ ID NO:59) |
| | | GTTRLYANASLGLFG (SEQ ID NO:69) |
| | | GTTRSYANASIGLFG (SEQ ID NO:84) |
| | | GTTRLYANASAGLFG (SEQ ID NO:40) |
| #107 | TGGFGSYVAFIPEKQ (SEQ ID NO:5) | TGGFGAYVAFIPEKQ (SEQ ID NO:48) |
| | | TGGFGSAVAFIPEKQ (SEQ ID NO:49) |
| | | TGGFGSYAAFIPEKQ (SEQ ID NO:50) |
| | | TGGFGSYVAFAPEKQ (SEQ ID NO:52) |
| | | TGGFGSYVAFIAEKQ (SEQ ID NO:53) |

## EXAMPLE 6

### Modifications to Peptide #49

As indicated above, specific amino acid substitutions in peptide #49 were tested in the I-MUNE® assay (see above) on an additional set of 69 donors along with the alanine scan mutagenized peptides. These peptides were tested as 15-mer peptides offset by 3 amino acids across the peptide sequence of beta-lactamase that encompasses epitope #49. These tests were performed in order to ensure that the amino acid variants did not introduce a *de novo* CD4+ T-cell epitope in another frame.

The assay was conducted on the following set of peptides listed in Table 8:

**Table 8. Peptide #49 Parent Series GTTRLYANASIGLFG (SEQ ID NO:4)**

| Peptide # | Sequence | SEQ ID NO: |
|---|---|---|
| 1 | WKPGTTRLYANASIG | 91 |
| 2 | GTTRLYANASIGLFG | 4 |
| 3 | RLYANASIGLFGALA | 92 |
| 4 | ANASIGLFGALAVKP | 93 |
| 5 | SIGLFGALAVKPSGN | 57 |

The results for these peptides are provided in Figure 6. In this Figure, each peptide number corresponds to the respective peptides in Table 8. The parent peptide is indicated in Table 8 and Figure 6 as peptide #2.

The assay was also conducted on the following set of peptides, in which the starting (*i.e.*, the modified epitope) has the substitution I155F.

**Table 9. Peptide #49 Series GTTRLYANASFGLFG (SEQ ID NO:59)**

| Peptide # | Sequence | SEQ ID NO: |
|---|---|---|
| 1 | WKPGTTRLYANASFG | 58 |
| 2 | GTTRLYANASFGLFG | 59 |
| 3 | RLYANASFGLFGALA | 60 |
| 4 | ANASFGLFGALAVKP | 61 |
| 5 | SFGLFGALAVKPSGN | 62 |

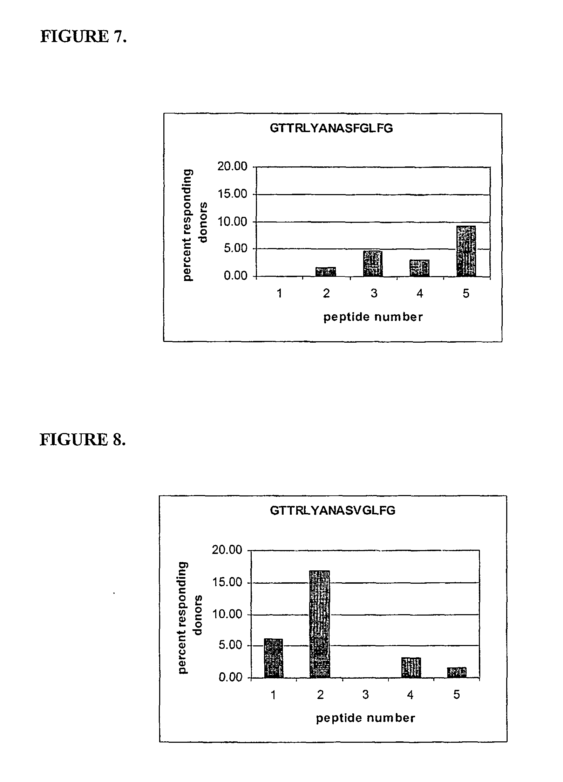

The results for these peptides are provided in Figure 7. In this Figure, each peptide number corresponds to the respective peptides in Table 9. The modified epitope is indicated in Table 9 and Figure 7 as peptide #2.

The assay was also conducted on the following set of peptides, in which the starting (*i.e.*, the modified epitope) has the substitution I155V.

**Table 10. Peptide #49 Series GTTRLYANASVGLFG (SEQ ID NO:63)**

| Peptide # | Sequence | SEQ ID NO: |
|---|---|---|
| 1 | WKPGTTRLYANASVG | 64 |
| 2 | GTTRLYANASVGLFG | 65 |
| 3 | RLYANASVGLFGALA | 66 |
| 4 | ANASYGLFGALAVKP | 67 |
| 5 | SVGLFGALAVKPSGN | 68 |

The results for these peptides are provided in Figure 8. In this Figure, each peptide number corresponds to the respective peptides in Table 10. The modified epitope is indicated in Table 10 and Figure 8 as peptide #2.

The assay was also conducted on the following set of peptides, in which the starting (*i.e.*, the modified epitope) has the substitution I155L.

**Table 11. Peptide #49 Series GTTRLYANASLGLFG (SEQ ID NO:69)**

| Peptide # | Sequence | SEQ ID NO: |
|---|---|---|
| 1 | WKPGTTRLYANASLG | 70 |
| 2 | GTTRLYANASLGLFG | 71 |
| 3 | RLYANASLGLFGALA | 72 |
| 4 | ANASLGLFGALAVKP | 73 |
| 5 | SLGLFGALAVKPSGN | 74 |

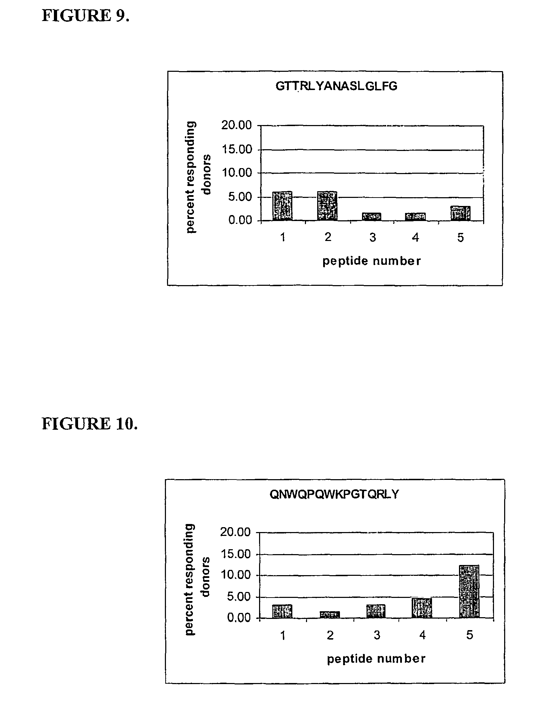

The results for these peptides are provided in Figure 9. In this Figure, each peptide number corresponds to the respective peptides in Table 11. The modified epitope is indicated in Table 11 and Figure 9 as peptide #2.

As indicated in Figures 7-9, of these three changes, the I155V change increased the percent of responders to the modified epitope sequence. The I155F and I155L changes had little effect.

Three additional changes in epitope #49 were tested, T147Q, L149S and L149R. As shown in Figures 10-12, only L149S had an effect on the epitope response rate. These peptides were also tested as 3-mer offsets, as described above.

Thus, the assay was also conducted on the following set of peptides, in which the starting (*i.e.,* modified epitope) has the substitution T147Q.

**Table 12. Peptide #49 Series QNWQPQWKPGTQRLY (SEQ ID NO:75)**

| Peptide # | Sequence | SEQ ID NO: |
|---|---|---|
| 1 | RFYQNWQPQWKPGTQ | 76 |
| 2 | QNWQPQWKPGTQRLY | 77 |
| 3 | QPQWKPGTQRLYANA | 78 |
| 4 | WKPGTQRLYANASIG | 79 |
| 5 | GTQRLYANASIGLFG | 80 |

The results for these peptides are provided in Figure 10. In this Figure, each peptide number corresponds to the respective peptides in Table 12. The modified epitope is indicated in Table 12 and Figure 10 as peptide #5.

The assay was also conducted on the following set of peptides, in which the starting (*i.e.*, the modified epitope) has the substitution L149S.

**Table 13. Peptide #49 Series QPQWKPGTTRSYANA (SEQ ID NO:82)**

| Peptide # | Sequence | SEQ ID NO: |
|---|---|---|
| 1 | QNWQPQWKPGTTRSY | 81 |
| 2 | QPQWKPGTTRSYANA | 82 |
| 3 | WKPGTTRSYANASIG | 83 |

(continued)

| Peptide # | Sequence | SEQ ID NO: |
|-----------|----------|------------|
| 4 | GTTRSYANASIGLFG | 84 |
| 5 | RSYANASIGLFGALA | 85 |

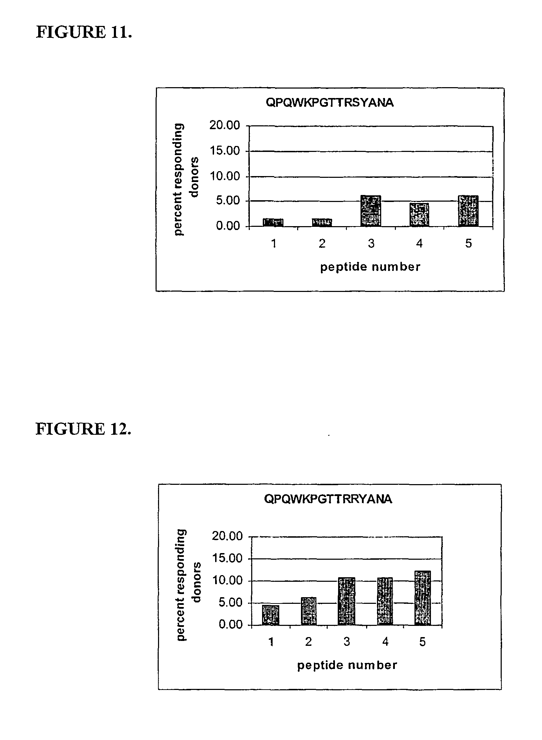

The results for these peptides are provided in Figure 11. In this Figure, each peptide number corresponds to the respective peptides in Table 12. The parent peptide is indicated in Table 12 and Figure 11 as peptide #4.

The assay was also conducted on the following set of peptides, in which the starting (*i.e.*, "parent" peptide) has the substitution L149R.

**Table 14. Peptide #49 Series QPQWKPGTTRRYANA (SEQ ID NO:87)**

| Peptide # | Sequence | SEQ ID NO: |
|-----------|----------|------------|
| 1 | QNWQPQWKPGTTRRY | 86 |
| 2 | QPQWKPGTTRRYANA | 87 |
| 3 | WKPGTTRRYANASIG | 88 |
| 4 | GTTRRYANASIGLFG | 89 |
| 5 | RRYANASIGLFGALA | 90 |

The results for these peptides are provided in Figure 12. In this Figure, each peptide number corresponds to the respective peptides in Table 14. The modified epitope is indicated in Table 14 and Figure 12 as peptide #4.

## EXAMPLE 7

### PBMC Proliferation Assay

In this Example, experiments conducted to assess the ability of beta-lactamase and epitope-modified beta-lactamase to stimulate PBMCs are described. All of the proteins were purified to approximately 2 mg/ml.

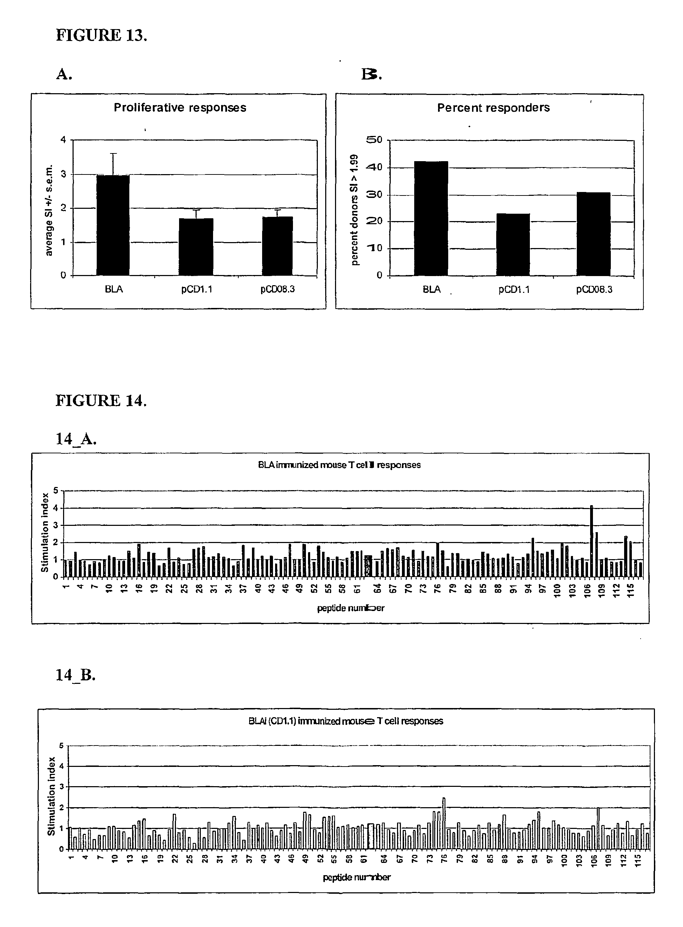

The blood samples used in these experiments were the same as described above (*i.e.*, before Example 1). The PBMCs were separated using Lymphoprep, as known in the art. The PBMCs were washed in PBS and counted using a Cell Dyn® 3700 blood analyzer (Abbott). The cell numbers and differentials were recorded. The PBMCs were resuspended to $4 \times 10^6$ cells/ml, in a solution of heat-inactivated human AB serum, RPMI 1640, pen/strep, glutamine, and 2-ME. Then, 2 mls per well were plated into 24-well plates. Two wells were used as no-enzyme controls. Then, the unmodified beta-lactamase and modified beta-lactamases were added to the wells at a concentrations of 10 ug/ml, 20 ug/ml, and 40 ug/ml. The epitope-modified beta-lactamases tested were K21A/S324A (designated as "pCD1.1") and K21A/S324A/L149S (designated as "pCD08.3"). The K21A mutation corresponds to SEQ ID NO:11, while the S324A mutation corresponds to SEQ ID NO:48, and the L149S mutation corresponds to SEQ ID NO:84.The S324 variant is in epitope #107, while K21A is in epitope #6, and L149S is in epitope #49. The plates were incubated at 37°C, in a 5% $CO_2$, humidified atmosphere for 6-7 days. On the day of harvest, the cells in each well were mixed and resuspended in the wells. Then, 8 aliquots of 100 ul from each well were transferred to a 96-well microtiter plate. To these wells, 0.25 uCi of tritiated thymidine were added. These plates were incubated for 6 hours, the cells harvested and counted. For analysis, the data for the eight replicates from each well were averaged. For the controls, the two wells were sampled to provide a total of 32 replicates. Each set of eight control wells was averaged, and the four average values were used to calculate a CV for each donor. SI values were calculated by dividing the average for each set of eight wells for each sample by the average CPM for the control well. The data were analyzed by creating a dataset representing the highest SI value achieved for each donor and each enzyme. A donor was considered to have responded if the highest SI value was greater than 1.99. A total of 26 donors were tested; the results are shown in Figure 13, with the average SI in Panel A and the percent responders in Panel B.

The results indicated that both of these epitope-modified beta-lactamases (pCD1.1 and pCD08.3) induced less proliferation in fewer donors overall, as compared to the wild-type beta-lactamase. There was no difference between the two epitope-modified beta-lactamases, indicating that the modification at position 149 (L149S) did not contribute to an increased immunogenicity of beta-lactamase.

**EXAMPLE 8**

**Testing the Reduced Immunogenicity BLA variant, pCD1.1 in CB6F1 Mice**

In this Example, experiments conducted to test the reduced immunogenicity of pCD1.1 *in vivo* are described. In these experiments, CB6F1 mice were immunized intraperitoneally with 20 μg of either wild-type BLA, or the CD1.1 protein in alum per mouse, as known in the art. Mice were immunized on days 1, 7, and 15 (*i.e.*, d=1, d=7 and d=15). Splenocytes were tested for reactivity with BLA peptides on day 19. Figure 14 shows the proliferative response of mouse splenocytes from BLA or CD1.1 immunized mice to BLA peptides. As indicated by these data, splenocytes from mice immunized with BLA respond strongly to the region around peptide 107. This response correlates with one of the human defined epitopes. Responses were also noted to the peptide 114 region. Splenocytes from mice immunized with the CD1.1 protein no longer responded to the peptide 107 region, nor the 114 region. These results indicate that the S324A mutation in the 107 peptide affects the peptide's ability to induce proliferation.

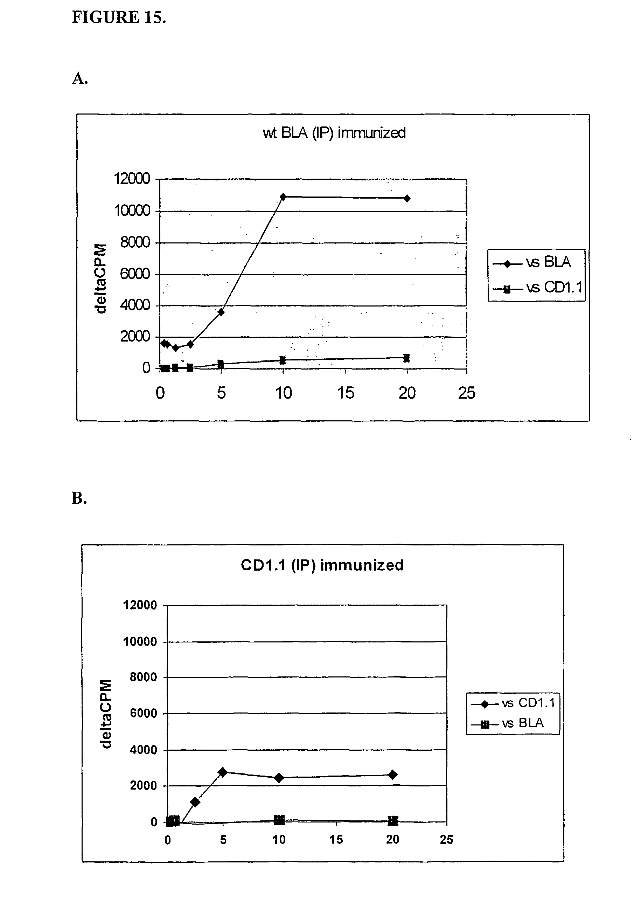

Splenocytes from immunized mice were tested *in vitro* for their proliferative response to whole protein BLA or CD1.1. The results are shown in Figure 15. With increasing BLA, the proliferative response by BLA-immunized mouse splenocytes was found to be increased. Splenocytes from mice immunized with CD1.1 protein were unable to respond significantly *in vitro* to either the epitope-intact parent protein, BLA, or the epitope modified CD1.1 protein. These results indicate that the S324A modification in peptide 107 has severely attenuated the immunogenicity of the protein. It is contemplated that the low level of response to CD1.1 in this experiment represented proliferation directed at peptide #75, a new T cell epitope revealed by the S324A modification. However, it is not intended that the present invention be limited to any particular mechanism.

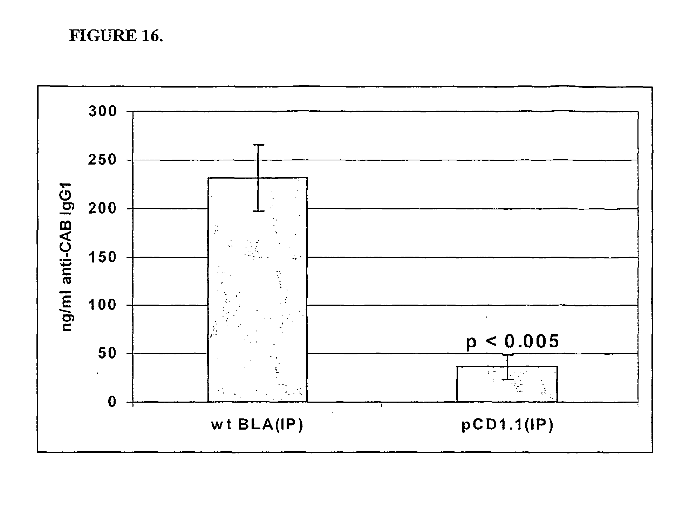

In addition, serum samples were taken from the thrice-immunized mice and an antigen-specific ELISA was performed (Figure 16). Mice immunized with BLA produced robust levels of anti-BLA IgGl antibody. However, mice immunized with CD1.1 protein did not produce significant levels of BLA-specific IgG1 antibody, as detected by this assay system. ELISA assays were performed using both BLA and CD1.1 as the plate-coating reagent; with identical results.

These results show that attenuating a proliferative response to an immunodominant epitope in mice results in the loss of immunogenicity of the whole protein at both the T cell proliferation and antibody production level. The level of proliferation seen to the CD1.1 protein that suggested a new epitope response was not enough to induce antibody responses in this immunization protocol. The epitope targeted was identical to an immunodominant epitope identified in the human population.

Although the invention has been described in connection with specific preferred embodiments, it should be understood that the invention should not be unduly limited to such specific embodiments. Indeed, various modifications of the described modes for carrying out the invention which that are obvious to those skilled in molecular biology, immunology, formulations, and/or related fields are intended to be within the scope of the present invention.

**Claims**

1. An isolated variant microbial beta-lactamase, which is a variant of a precursor beta-lactamase from *Enterobacter cloacae*, the variant having an amino acid sequence comprising at least one modification of an amino acid made at a position in the precursor which corresponds in position in primary or tertiary structure to a position in an epitope defined by SEQ ID NO: 2, SEQ ID NO: 3, SEQ ID NO: 4 or SEQ ID NO: 5 of *E. cloacae* beta-lactamase having the amino acid sequence of SEQ ID NO: 1, whereby the immunogenic response produced by said variant beta-lactamase is less than the immunogenic response produced by said precursor beta-lactamase,
   wherein said variant beta-lactamase has at least 80% amino acid sequence identity to said precursor beta-lactamase.

2. The isolated variant beta-lactamase of claim 1 wherein said variant beta-lactamase has at least 90% amino acid sequence identity to said precursor beta-lactamase.

3. A method of reducing immunogenicity of a precursor microbial beta-lactamase from *Enterobacter cloacae*, comprising modifying at least one amino acid at a position which corresponds in position in primary or tertiary structure to a position in an epitope defined by SEQ ID NO: 2, SEQ ID NO: 3, SEQ ID NO: 4 or SEQ ID NO: 5 of *E. cloacae* beta-lactamase having the amino acid sequence of SEQ ID NO: 1 to produce a variant beta-lactamase, whereby said variant beta-lactamase is less immunogenic than the precursor beta-lactamase.

4. The isolated variant beta-lactamase of claim 1 or method of claim 3 wherein said precursor beta-lactamase is modified by addition, deletion or substitution of at least one amino acid in said T cell epitope.

5. The isolated variant beta-lactamase of claim 1 or method of claim 3 wherein the number of differing amino acids between said precursor beta-lactamase and said variant beta-lactamase is between 1 and 10.

6. The isolated variant beta-lactamase of claim 1 or method of claim 3 wherein said precursor beta-lactamase is modified by substituting the amino acid sequence of said T-cell epitope with an analogous sequence from a homolog of said precursor beta-lactamase, wherein said substitution mimics the tertiary structure of said T-cell epitope.

7. The isolated variant beta-lactamase of claim 1 or method of claim 3, wherein said variant beta-lactamase comprises at least one epitope comprising an amino acid sequence selected from the group consisting of SEQ ID NOS: 10, 11, 20, 21, 25, 40, 48, 49, 50, 52, 53, 59, 69 and 84.

8. The isolated variant beta-lactamase of claim 1 or method of claim 3 wherein said precursor microbial beta-lactamase has the sequence of SEQ ID NO: 1.

9. A composition comprising the beta-lactamase of claim 1.

10. An isolated nucleic acid encoding the beta-lactamase of claim 1.

11. An expression vector comprising a polynucleotide sequence encoding the beta-lactamase of Claim 1.

12. A host cell comprising said expression vector of claim 11.

13. A method for reducing the immunogenicity of a microbial beta-lactamase from *Enterobacter cloacae* comprising the steps of :

   (a) identifying at least one T-cell epitope in the beta-lactamase by:

   (i) contacting an adherent monocyte-derived dendritic cell that has been differentiated by exposure to at least one cytokine in vitro, with at least one peptide comprising said T-cell epitope; and
   (ii) contacting said dendritic cell and said peptide with a naive T-cell, wherein said naive T-cell has been obtained from the same source as said adherent monocyte-derived dendritic cell, and whereby the T-cell proliferates in response to said peptide; and

   (b) modifying said beta-lactamase to neutralize said T-cell epitope to produce a variant beta-lactamase, such that said variant beta-lactamase induces less than or substantially equal to the baseline proliferation of said naive T-cells;

   wherein said epitope of said beta-lactamase is modified by substituting the amino acid sequence of said T-cell epitope with an analogous sequence from a homolog of said beta-lactamase, wherein said substitution mimics the tertiary structure of said T-cell epitope, and wherein said epitope is selected from the group consisting of SEQ ID NOS: 2, 3, 4, and 5.

14. A peptide comprising an amino acid sequence selected from the group consisting of SEQ ID NOs: 10, 11, 20, 21, 25, 40, 48, 49, 50, 52, 53, 59, 69 and 84.

15. An isolated beta-lactamase having the sequence:

```
Thr Pro Val Ser Glu Lys Gln Leu Ala Glu Val Val Ala Asn Thr Ile
 1               5                10                   15
Thr Pro Leu Met Ala Ala Gln Ser Val Pro Gly Met Ala Val Ala Val
         20                  25                   30
Ile Tyr Gln Gly Lys Pro His Tyr Tyr Thr Phe Gly Lys Ala Asp Ile
         35                  40                   45
Ala Ala Asn Lys Pro Val Thr Pro Gln Thr Leu Phe Glu Leu Gly Ser
         50                  55                   60
Ile Ser Lys Thr Phe Thr Gly Val Leu Gly Gly Asp Ala Ile Ala Arg
 65              70                   75                   80
Gly Glu Ile Ser Leu Asp Asp Ala Val Thr Arg Tyr Trp Pro Gln Leu
             85                  90                   95
Thr Gly Lys Gln Trp Gln Gly Ile Arg Met Leu Asp Leu Ala Thr Tyr
         100                 105                 110
Thr Ala Gly Gly Leu Pro Leu Gln Val Pro Asp Glu Val Thr Asp Asn
         115                 120                 125
Ala Ser Leu Leu Arg Phe Tyr Gln Asn Trp Gln Pro Gln Trp Lys Pro
         130             135                 140
Gly Thr Thr Arg Leu Tyr Ala Asn Ala Ser Ile Gly Leu Phe Gly Ala
 145             150                 155                 160
Leu Ala Val Lys Pro Ser Gly Met Pro Tyr Glu Gln Ala Met Thr Thr
             165                 170                 175
Arg Val Leu Lys Pro Leu Lys Leu Asp His Thr Trp Ile Asn Val Pro
             180                 185                 190
Lys Ala Glu Glu Ala His Tyr Ala Trp Gly Tyr Arg Asp Gly Lys Ala
         195                 200                 205
Val Arg Val Ser Pro Gly Met Leu Asp Ala Gln Ala Tyr Gly Val Lys
     210                 215                 220
Thr Asn Val Gln Asp Met Ala Asn Trp Val Met Ala Asn Met Ala Pro
 225                 230                 235                 240
Glu Asn Val Ala Asp Ala Ser Leu Lys Gln Gly Ile Ala Leu Ala Gln
             245                 250                 255
Ser Arg Tyr Trp Arg Ile Gly Ser Met Tyr Gln Gly Leu Gly Trp Glu
             260                 265                 270
Met Leu Asn Trp Pro Val Glu Ala Asn Thr Val Val Glu Gly Ser Asp
         275                 280                 285
Ser Lys Val Ala Leu Ala Pro Leu Pro Val Ala Glu Val Asn Pro Pro
         290                 295                 300
Ala Pro Pro Val Lys Ala Ser Trp Val His Lys Thr Gly Ser Thr Gly
 305                 310                 315                 320
Gly Phe Gly Ala Tyr Val Ala Phe Ile Pro Glu Lys Gln Ile Gly Ile
             325                 330                 335
Val Met Leu Ala Asn Thr Ser Tyr Pro Asn Pro Ala Arg Val Glu Ala
         340                 345                 350
Ala Tyr His Ile Leu Glu Ala Leu Gln
         355                 360
```

16. An isolated beta-lactamase having the sequence:

```
Thr Pro Val Ser Glu Lys Gln Leu Ala Glu Val Val Ala Asn Thr Ile
 1               5                10                   15
Thr Pro Leu Met Ala Ala Gln Ser Val Pro Gly Met Ala Val Ala Val
         20                  25                   30
Ile Tyr Gln Gly Lys Pro His Tyr Tyr Thr Phe Gly Lys Ala Asp Ile
         35                  40                   45
Ala Ala Asn Lys Pro Val Thr Pro Gln Thr Leu Phe Glu Leu Gly Ser
```

```
            50                      55                      60
Ile Ser Lys Thr Phe Thr Gly Val Leu Gly Gly Asp Ala Ile Ala Arg
65                      70                      75                      80
Gly Glu Ile Ser Leu Asp Asp Ala Val Thr Arg Tyr Trp Pro Gln Leu
                85                      90                      95
Thr Gly Lys Gln Trp Gln Gly Ile Arg Met Leu Asp Leu Ala Thr Tyr
            100                     105                     110
Thr Ala Gly Gly Leu Pro Leu Gln Val Pro Asp Glu Val Thr Asp Asn
            115                     120                     125
Ala Ser Leu Leu Arg Phe Tyr Gln Asn Trp Gln Pro Gln Trp Lys Pro
            130                     135                     140
Gly Thr Thr Arg Ser Tyr Ala Asn Ala Ser Ile Gly Leu Phe Gly Ala
145                     150                     155                     160
Leu Ala Val Lys Pro Ser Gly Met Pro Tyr Glu Gln Ala Met Thr Thr
                165                     170                     175
Arg Val Leu Lys Pro Leu Lys Leu Asp His Thr Trp Ile Asn Val Pro
                180                     185                     190
Lys Ala Glu Glu Ala His Tyr Ala Trp Gly Tyr Arg Asp Gly Lys Ala
            195                     200                     205
Val Arg Val Ser Pro Gly Met Leu Asp Ala Gln Ala Tyr Gly Val Lys
210                     215                     220
Thr Asn Val Gln Asp Met Ala Asn Trp Val Met Ala Asn Met Ala Pro
225                     230                     235                     240
Glu Asn Val Ala Asp Ala Ser Leu Lys Gln Gly Ile Ala Leu Ala Gln
            245                     250                     255
Ser Arg Tyr Trp Arg Ile Gly Ser Met Tyr Gln Gly Leu Gly Trp Glu
            260                     265                     270
Met Leu Asn Trp Pro Val Glu Ala Asn Thr Val Val Glu Gly Ser Asp
            275                     280                     285
Ser Lys Val Ala Leu Ala Pro Leu Pro Val Ala Glu Val Asn Pro Pro
            290                     295                     300
Ala Pro Pro Val Lys Ala Ser Trp Val His Lys Thr Gly Ser Thr Gly
305                     310                     315                     320
Gly Phe Gly Ala Tyr Val Ala Phe Ile Pro Glu Lys Gln Ile Gly Ile
                325                     330                     335
Val Met Leu Ala Asn Thr Ser Tyr Pro Asn Pro Ala Arg Val Glu Ala
            340                     345                     350
Ala Tyr His Ile Leu Glu Ala Leu Gln
            355                     360
```

## Patentansprüche

1. Isolierte mikrobielle β-Lactamase-Variante, die eine Variante eines β-Lactamase-Vorläufers aus Enterobacter cloacae ist, wobei die Variante eine Aminosäuresequenz aufweist, die zumindest eine Modifikation einer Aminosäure umfasst, die an einer Position im Vorläufer durchgeführt wurde, die in ihrer Position in der Primär-oder Tertiärstruktur einer Position in einem von Seq.-ID Nr. 2, Seq.-ID Nr. 3, Seq.-ID Nr. 4 oder Seq.-ID Nr. 5 definierten Epitop von E.-cloacae-β-Lactamase entspricht, das die Aminosäuresequenz von Seq.-ID Nr. 1 aufweist, wodurch die immunogene Reaktion, die von der β-Lactamase-Variante produziert wird, schwächer ist als die immunogene Reaktion, die vom β-Lactamase-Vorläufer produziert wird, worin die β-Lactamase-Variante zumindest 80%ige Aminosäuresequenzidentität mit dem β-Lactamase-Vorläufer aufwe! ist.

2. Isolierte β-Lactamase-Variante nach Anspruch 1, worin die β-Lactamase-Variante zumindest 90%ige Aminosäuresequenzidentität mit dem β-Lactamase-Vorläufer aufweist.

3. Verfahren zum Verringern der Immunogenität eines mikrobiellen β-Lactamase-Vorläufers aus Enterobacter cloacae, umfassend das Modifizieren zumindest einer Aminosäure an einer Position, die in ihrer Position in der Primär- oder Tertiärstruktur einer Position in einem von Seq.-ID Nr. 2, Seq.-ID Nr. 3, Seq.-ID Nr. 4 oder Seq.-ID Nr. 5 definierten Epitop von E.-cloacae-β-Lactamase entspricht, das die Aminosäuresequenz von Seq.-ID Nr. 1 aufweist, um eine β-Lactamase-Variante zu erzeugen, wodurch die β-Lactamase-Variante weniger immunogen ist als der β-Lactamase-Vorläufer.

**4.** Isolierte β-Lactamase-Variante nach Anspruch 1 oder Verfahren nach Anspruch 3, worin der β-Lactamase-Vorläufer durch Addition, Deletion oder Substitution zumindest einer Aminosäure im T-Zellen-Epitop modifiziert ist bzw. wird.

**5.** Isolierte β-Lactamase-Variante nach Anspruch 1 oder Verfahren nach Anspruch 3, worin die Anzahl der Aminosäuren, die im β-Lactamase-Vorläufer und in der β-Lactamase-Variante unterschiedlich sind, zwischen 1 und 10 liegt.

**6.** Isolierte β-Lactamase-Variante nach Anspruch 1 oder Verfahren nach Anspruch 3, worin der β-Lactamase-Vorläufer durch Substituieren der Aminosäuresequenz des T-Zellen-Epitops durch eine analoge Sequenz aus einem Homolog des β-Lactamase-Vorläufers modifiziert ist bzw. wird, worin die Substitution die Tertiärstruktur des T-Zellen-Epitops nachahmt.

**7.** Isolierte β-Lactamase-Variante nach Anspruch 1 oder Verfahren nach Anspruch 3, worin die β-Lactamase-Variante zumindest ein Epitop umfasst, das eine aus der aus den Seq.-ID Nr. 10, 11, 20, 21, 25, 40, 48, 49, 50, 52, 53, 59, 69 und 84 bestehenden Gruppe ausgewählte Aminosäuresequenz umfasst.

**8.** Isolierte β-Lactamase-Variante nach Anspruch 1 oder Verfahren nach Anspruch 3, worin der mikrobielle β-Lactamase-Vorläufer die Sequenz von Seq.-ID Nr. 1 aufweist.

**9.** Zusammensetzung, umfassend eine β-Lactamase nach Anspruch 1.

**10.** Isolierte Nucleinsäure, die für eine β-Lactamase nach Anspruch 1 kodiert.

**11.** Expressionsvektor, umfassend eine Polynucleotidsequenz, die für eine β-Lactamase nach Anspruch 1 kodiert.

**12.** Wirtszelle, umfassend einen Expressionsvektor nach Anspruch 11.

**13.** Verfahren zum Verringern der Immunogenität einer mikrobiellen β-Lactamase aus Enterobacter cloacae, umfassend folgende Schritte:

(a) das Identifizieren zumindest eines T-Zellen-Epitops in der β-Lactamase durch:

(i) Kontaktieren einer adhärenten, monozytenderivierten dendritischen Zelle, die durch Aussetzen gegenüber zumindest einem Zytokin in vitro differenziert wurde, mit zumindest einem Peptid, das das T-Zellen-Epitop umfasst; und
(ii) Kontaktieren der dendritischen Zelle und des Peptids mit einer naiven T-Zelle, worin die naive T-Zelle aus derselben Quelle erhalten wurde wie die adhärente, monozytenderivierte dendritische Zelle, und wodurch die T-Zelle als Reaktion auf das Peptid proliferiert; und

(b) das Modifizieren der β-Lactamase, um das T-Zellen-Epitop zu neutralisieren und eine β-Lactamase-Variante zu erzeugen, so dass die β-Lactamase-Variante weniger oder im Wesentlichen gleich viel Proliferation als/wie die Grundlinien-Proliferation der nativen T-Zellen induziert,

worin das Epitop der β-Lactamase durch Substituieren der Aminosäuresequenz des T-Zellen-Epitops mit einer analogen Sequenz aus einem Homolog des β-Lactamase-Vorläufers modifiziert wird, worin die Substitution die Tertiärstruktur des T-Zellen-Epitops nachahmt und worin das Epitop aus der aus den Seq.-ID Nr. 2, 3, 4 und 5 bestehenden Gruppe ausgewählt wird.

**14.** Peptid, umfassend eine aus der aus den Seq.-ID Nr. 10, 11, 20, 21, 25, 40, 48, 49, 50, 52, 53, 59, 69 und 84 bestehenden Gruppe ausgewählte Aminosäuresequenz.

**15.** Isolierte β-Lactamase mit der Sequenz:

```
Thr Pro Val Ser Glu Lys Gln Leu Ala Glu Val Val Ala Asn Thr Ile
1               5               10              15
Thr Pro Leu Met Ala Ala Gln Ser Val Pro Gly Met Ala Val Ala Val
            20              25              30
Ile Tyr Gln Gly Lys Pro His Tyr Tyr Thr Phe Gly Lys Ala Asp Ile
            35              40              45
Ala Ala Asn Lys Pro Val Thr Pro Gln Thr Leu Phe Glu Leu Gly Ser
    50              55              60
Ile Ser Lys Thr Phe Thr Gly Val Leu Gly Gly Asp Ala Ile Ala Arg
65              70              75              80
Gly Glu Ile Ser Leu Asp Asp Ala Val Thr Arg Tyr Trp Pro Gln Leu
            85              90              95
Thr Gly Lys Gln Trp Gln Gly Ile Arg Met Leu Asp Leu Ala Thr Tyr
        100             105             110
Thr Ala Gly Gly Leu Pro Leu Gln Val Pro Asp Glu Val Thr Asp Asn
        115             120             125
Ala Ser Leu Leu Arg Phe Tyr Gln Asn Trp Gln Pro Gln Trp Lys Pro
    130             135             140
```

```
Gly Thr Thr Arg Leu Tyr Ala Asn Ala Ser Ile Gly Leu Phe Gly Ala
145             150             155             160
Leu Ala Val Lys Pro Ser Gly Met Pro Tyr Glu Gln Ala Met Thr Thr
            165             170             175
Arg Val Leu Lys Pro Leu Lys Leu Asp His Thr Trp Ile Asn Val Pro
        180             185             190
Lys Ala Glu Glu Ala His Tyr Ala Trp Gly Tyr Arg Asp Gly Lys Ala
        195             200             205
Val Arg Val Ser Pro Gly Met Leu Asp Ala Gln Ala Tyr Gly Val Lys
    210             215             220
Thr Asn Val Gln Asp Met Ala Asn Trp Val Met Ala Asn Met Ala Pro
225             230             235             240
Glu Asn Val Ala Asp Ala Ser Leu Lys Gln Gly Ile Ala Leu Ala Gln
            245             250             255
Ser Arg Tyr Trp Arg Ile Gly Ser Met Tyr Gln Gly Leu Gly Trp Glu
        260             265             270
Met Leu Asn Trp Pro Val Glu Ala Asn Thr Val Val Glu Gly Ser Asp
        275             280             285
Ser Lys Val Ala Leu Ala Pro Leu Pro Val Ala Glu Val Asn Pro Pro
    290             295             300
Ala Pro Pro Val Lys Ala Ser Trp Val His Lys Thr Gly Ser Thr Gly
305             310             315             320
Gly Phe Gly Ala Tyr Val Ala Phe Ile Pro Glu Lys Gln Ile Gly Ile
            325             330             335
Val Met Leu Ala Asn Thr Ser Tyr Pro Asn Pro Ala Arg Val Glu Ala
        340             345             350
Ala Tyr His Ile Leu Glu Ala Leu Gln
    355             360
```

**16.** Isolierte β-Lactamase mit der Sequenz:

```
Thr Pro Val Ser Glu Lys Gln Leu Ala Glu Val Val Ala Asn Thr Ile
 1           5               10              15
Thr Pro Leu Met Ala Ala Gln Ser Val Pro Gly Met Ala Val Ala Val
            20              25              30
Ile Tyr Gln Gly Lys Pro His Tyr Tyr Thr Phe Gly Lys Ala Asp Ile
        35              40              45
Ala Ala Asn Lys Pro Val Thr Pro Gln Thr Leu Phe Glu Leu Gly Ser
        50              55              60
Ile Ser Lys Thr Phe Thr Gly Val Leu Gly Gly Asp Ala Ile Ala Arg
65              70              75              80
Gly Glu Ile Ser Leu Asp Asp Ala Val Thr Arg Tyr Trp Pro Gln Leu
            85              90              95
Thr Gly Lys Gln Trp Gln Gly Ile Arg Met Leu Asp Leu Ala Thr Tyr
            100             105             110
Thr Ala Gly Gly Leu Pro Leu Gln Val Pro Asp Glu Val Thr Asp Asn
            115             120             125
Ala Ser Leu Leu Arg Phe Tyr Gln Asn Trp Gln Pro Gln Trp Lys Pro
        130             135             140
Gly Thr Thr Arg Ser Tyr Ala Asn Ala Ser Ile Gly Leu Phe Gly Ala
145             150             155             160
Leu Ala Val Lys Pro Ser Gly Met Pro Tyr Glu Gln Ala Met Thr Thr
            165             170             175
Arg Val Leu Lys Pro Leu Lys Leu Asp His Thr Trp Ile Asn Val Pro
            180             185             190
```

```
Lys Ala Glu Glu Ala His Tyr Ala Trp Gly Tyr Arg Asp Gly Lys Ala
        195             200             205
Val Arg Val Ser Pro Gly Met Leu Asp Ala Gln Ala Tyr Gly Val Lys
    210             215             220
Thr Asn Val Gln Asp Met Ala Asn Trp Val Met Ala Asn Met Ala Pro
225             230             235             240
Glu Asn Val Ala Asp Ala Ser Leu Lys Gln Gly Ile Ala Leu Ala Gln
            245             250             255
Ser Arg Tyr Trp Arg Ile Gly Ser Met Tyr Gln Gly Leu Gly Trp Glu
        260             265             270
Met Leu Asn Trp Pro Val Glu Ala Asn Thr Val Val Glu Gly Ser Asp
    275             280             285
Ser Lys Val Ala Leu Ala Pro Leu Pro Val Ala Glu Val Asn Pro Pro
    290             295             300
Ala Pro Pro Val Lys Ala Ser Trp Val His Lys Thr Gly Ser Thr Gly
305             310             315             320
Gly Phe Gly Ala Tyr Val Ala Phe Ile Pro Glu Lys Gln Ile Gly Ile
        325             330             335
Val Met Leu Ala Asn Thr Ser Tyr Pro Asn Pro Ala Arg Val Glu Ala
        340             345             350
Ala Tyr His Ile Leu Glu Ala Leu Gln
        355             360
```

## Revendications

1. Béta-lactamase microbienne variante isolée, qui est une variante d'une béta-lactamase précurseur de *Enterobacter cloacae*, la variante ayant une séquence d'acides aminés comprenant au moins une modification d'un acide aminé réalisée à une position dans le précurseur qui correspond en position à la structure primaire ou tertiaire à une position dans un épitope défini par la SEQ ID NO: 2, SEQ ID NO: 3, SEQ ID NO: 4 ou SEQ ID NO: 5 de E. *cloacae* béta-lactamase ayant la séquence d'acides aminés de la SEQ ID NO: 1, par quoi la réponse immunogénique produite par ladite béta-lactamase variante est inférieure à la réponse immunogénique produite par ladite béta-lactamase précurseur, où ladite béta-lactamase variante possède au moins 80% d'identité de séquence d'acides aminés avec ladite béta-lactamase précurseur.

2. Béta-lactamase variante isolée selon la revendication 1, où ladite béta-lactamase variante possède au moins 90% d'identité de séquence d'acides aminés avec ladite béta-lactamase précurseur.

3. Méthode pour réduire l'immunogénicité d'une béta-lactamase microbienne précurseur de *Enterobacter cloaceae*, comprenant la modification d'au moins un acide aminé à une position qui correspond en position dans la structure primaire ou tertiaire à une position dans un épitope défini par la SEQ ID NO: 2, SEQ ID NO: 3, SEQ ID NO: 4 ou SEQ ID NO: 5 de *E. cloacae* béta-lactamase ayant la séquence d'acides aminés de SEQ ID NO: 1 pour produire une béta-lactamase variante, par quoi ladite béta-lactamase variante est moins immunogène que la béta-lactamase précurseur.

4. Béta-lactamase variante isolée selon la revendication 1 ou méthode selon la revendication 3, où ladite béta-lactamase précurseur est modifiée par addition, délétion ou substitution d'au moins un acide aminé dans ledit épitope à cellule T.

5. Béta-lactamase variante isolée selon la revendication 1 ou méthode selon la revendication 3, où le nombre d'acides aminés différents entre ladite béta-lactamase précurseur et ladite béta-lactamase variante est entre 1 et 10.

6. Béta-lactamase variante isolée selon la revendication 1 ou méthode selon la revendication 3, où ladite béta-lactamase précurseur est modifiée en substituant la séquence d'acides aminés dudit épitope à cellule T par une séquence analogue d'un homologue de ladite béta-lactamase précurseur, où ladite substitution imite la structure tertiaire dudit épitope à cellule T.

7. Béta-lactamase variante isolée selon la revendication 1 ou méthode selon la revendication 3, où ladite béta-lactamase variante comprend au moins un épitope comprenant une séquence d'acides aminés sélectionnée dans le groupe consistant en SEQ ID NOS: 10, 11, 20, 21, 25, 40, 48, 49, 50, 52, 53, 59, 69 et 84.

8. Béta-lactamase variante isolée selon la revendication 1 ou méthode selon la revendication 3, où ladite béta-lactamase microbienne précurseur présente la séquence de SEQ ID NO: 1.

9. Composition comprenant la béta-lactamase de la revendication 1.

10. Acide nucléique isolé codant pour la béta-lactamase de la revendication 1.

11. Vecteur d'expression comprenant une séquence de polynucléotides codant pour la béta-lactamase de la revendication 1.

12. Cellule hôte comprenant ledit vecteur d'expression de la revendication 11.

13. Méthode pour réduire l'immunogénicité d'une béta-lactamase microbienne de *Enterobacter cloacae* comprenant les étapes

(a) identifier au moins un épitope à cellule-T dans la béta-lactamase en:

(i) mettant en contact une cellule dendritique adhérente dérivée d'un monocyte qui a été différencié par exposition à au moins une cytokine in vitro, avec au moins un peptide comprenant ledit épitope à cellule-T; et
(ii) mettant en contact ladite cellule dendritique et ledit peptide avec une cellule-T naïve, où ladite cellule-T naïve a été obtenue de la même source que ladite cellule dendritique adhérente dérivée d'un monocyte, et par quoi la cellule-T prolifère en réponse audit peptide; et

(b) modifier ladite béta-lactamase pour neutraliser ledit épitope à cellule-T afin de produire une béta-lactamse variante de telle sorte que ladite béta-lactamase variante induit moins que ou d'une manière sensiblement égale à la prolifération de ligne de base desdites cellules-T naïves;

où ledit épitope de ladite béta-lactamase est modifiée en substituant la séquence d'acides aminés dudit épitope à cellule-T par une séquence analogue d'un homologue de ladite béta-lactamase, où ladite substitution imite la structure tertiaire dudit épitope à cellule-T, et où ledit épitope est sélectionné dans le groupe consistant en SEQ ID Nos: 2, 3, 4 et 5.

14. Peptide comprenant une séquence d'acides aminés sélectionnée dans le groupe consistant en SEQ ID Nos: 10, 11, 20, 21, 25, 40, 48, 49, 50, 52, 53, 59, 69 et 84.

15. Une Béta-lactamase isolée ayant la séquence:

```
Thr Pro Val Ser Glu Lys Gln Leu Ala Glu Val Val Ala Asn Thr Ile
 1               5                10                   15
Thr Pro Leu Met Ala Ala Gln Ser Val Pro Gly Met Ala Val Ala Val
             20                25                30
Ile Tyr Gln Gly Lys Pro His Tyr Tyr Thr Phe Gly Lys Ala Asp Ile
         35                40                45
Ala Ala Asn Lys Pro Val Thr Pro Gln Thr Leu Phe Glu Leu Gly Ser
     50                55                60
Ile Ser Lys Thr Phe Thr Gly Val Leu Gly Gly Asp Ala Ile Ala Arg
65                70                75                      80
Gly Glu Ile Ser Leu Asp Asp Ala Val Thr Arg Tyr Trp Pro Gln Leu
             85                90                      95
Thr Gly Lys Gln Trp Gln Gly Ile Arg Met Leu Asp Leu Ala Thr Tyr
         100               105               110
Thr Ala Gly Gly Leu Pro Leu Gln Val Pro Asp Glu Val Thr Asp Asn
         115               120               125
```

```
Ala Ser Leu Leu Arg Phe Tyr Gln Asn Trp Gln Pro Gln Trp Lys Pro
    130                 135             140
Gly Thr Thr Arg Leu Tyr Ala Asn Ala Ser Ile Gly Leu Phe Gly Ala
145             150             155                 160
Leu Ala Val Lys Pro Ser Gly Met Pro Tyr Glu Gln Ala Met Thr Thr
            165             170             175
Arg Val Leu Lys Pro Leu Lys Leu Asp His Thr Trp Ile Asn Val Pro
            180             185             190
Lys Ala Glu Glu Ala His Tyr Ala Trp Gly Tyr Arg Asp Gly Lys Ala
        195             200             205
Val Arg Val Ser Pro Gly Met Leu Asp Ala Gln Ala Tyr Gly Val Lys
    210             215             220
Thr Asn Val Gln Asp Met Ala Asn Trp Val Met Ala Asn Met Ala Pro
225             230             235             240
Glu Asn Val Ala Asp Ala Ser Leu Lys Gln Gly Ile Ala Leu Ala Gln
            245             250             255
Ser Arg Tyr Trp Arg Ile Gly Ser Met Tyr Gln Gly Leu Gly Trp Glu
            260             265             270
Met Leu Asn Trp Pro Val Glu Ala Asn Thr Val Val Glu Gly Ser Asp
        275             280             285
Ser Lys Val Ala Leu Ala Pro Leu Pro Val Ala Glu Val Asn Pro Pro
        290             295             300
Ala Pro Pro Val Lys Ala Ser Trp Val His Lys Thr Gly Ser Thr Gly
305             310             315             320
Gly Phe Gly Ala Tyr Val Ala Phe Ile Pro Glu Lys Gln Ile Gly Ile
            325             330             335
Val Met Leu Ala Asn Thr Ser Tyr Pro Asn Pro Ala Arg Val Glu Ala
            340             345             350
Ala Tyr His Ile Leu Glu Ala Leu Gln
        355             360
```

**16.** Une Béta-lactamase isolée ayant la séquence:

```
Thr Pro Val Ser Glu Lys Gln Leu Ala Glu Val Val Ala Asn Thr Ile
1           5               10                  15
Thr Pro Leu Met Ala Ala Gln Ser Val Pro Gly Met Ala Val Ala Val
        20              25                  30
Ile Tyr Gln Gly Lys Pro His Tyr Tyr Thr Phe Gly Lys Ala Asp Ile
        35              40                  45
Ala Ala Asn Lys Pro Val Thr Pro Gln Thr Leu Phe Glu Leu Gly Ser
    50              55                  60
Ile Ser Lys Thr Phe Thr Gly Val Leu Gly Gly Asp Ala Ile Ala Arg
65              70                  75                      80
Gly Glu Ile Ser Leu Asp Asp Ala Val Thr Arg Tyr Trp Pro Gln Leu
            85                  90                      95
Thr Gly Lys Gln Trp Gln Gly Ile Arg Met Leu Asp Leu Ala Thr Tyr
        100                 105                 110
Thr Ala Gly Gly Leu Pro Leu Gln Val Pro Asp Glu Val Thr Asp Asn
        115                 120                 125
Ala Ser Leu Leu Arg Phe Tyr Gln Asn Trp Gln Pro Gln Trp Lys Pro
    130             135                 140
Gly Thr Thr Arg Ser Tyr Ala Asn Ala Ser Ile Gly Leu Phe Gly Ala
145             150                 155                     160
Leu Ala Val Lys Pro Ser Gly Met Pro Tyr Glu Gln Ala Met Thr Thr
            165                 170                     175
Arg Val Leu Lys Pro Leu Lys Leu Asp His Thr Trp Ile Asn Val Pro
            180                 185                     190
```

```
Lys Ala Glu Glu Ala His Tyr Ala Trp Gly Tyr Arg Asp Gly Lys Ala
        195                 200                 205
Val Arg Val Ser Pro Gly Met Leu Asp Ala Gln Ala Tyr Gly Val Lys
    210                 215                 220
Thr Asn Val Gln Asp Met Ala Asn Trp Val Met Ala Asn Met Ala Pro
225                 230                 235                 240
Glu Asn Val Ala Asp Ala Ser Leu Lys Gln Gly Ile Ala Leu Ala Gln
            245                 250                 255
Ser Arg Tyr Trp Arg Ile Gly Ser Met Tyr Gln Gly Leu Gly Trp Glu
            260                 265                 270
Met Leu Asn Trp Pro Val Glu Ala Asn Thr Val Val Glu Gly Ser Asp
        275                 280                 285
Ser Lys Val Ala Leu Ala Pro Leu Pro Val Ala Glu Val Asn Pro Pro
    290                 295                 300
Ala Pro Pro Val Lys Ala Ser Trp Val His Lys Thr Gly Ser Thr Gly
305                 310                 315                 320
Gly Phe Gly Ala Tyr Val Ala Phe Ile Pro Glu Lys Gln Ile Gly Ile
            325                 330                 335
Val Met Leu Ala Asn Thr Ser Tyr Pro Asn Pro Ala Arg Val Glu Ala
            340                 345                 350
Ala Tyr His Ile Leu Glu Ala Leu Gln
            355                 360
```

FIGURE 1.

FIGURE 2.

F IGURE 3.

BLA Variants, Peptide #36

FIGURE 4.

BLA Variants, Peptide 49

FIGURE 5.

FIGURE 6.

FIGURE 7.

**GTTRLYANASFGLFG**

FIGURE 8.

**GTTRLYANASVGLFG**

FIGURE 9.

GTTRLYANASLGLFG

FIGURE 10.

QNWQPQWKPGTQRLY

## FIGURE 11.

**QPQWKPGTTRSYANA**

## FIGURE 12.

**QPQWKPGTTRRYANA**

## FIGURE 13.

**A.**

**B.**

Proliferative responses

Percent responders

## FIGURE 14.

### 14_A.

BLA immunized mouse T cell responses

### 14_B.

BLAi (CD1.1) immunized mouse T cell responses

## FIGURE 15.

A.

wt BLA (IP) immunized

B.

CD1.1 (IP) immunized

FIGURE 16.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2002047717 A
- US 4760025 A
- US RE34606 E
- US 5264366 A
- US 4683195 A
- US 4683202 A
- US 4965188 A

### Non-patent literature cited in the description

- **Singleton ; Sainsbury.** Dictionary of Microbiology and Molecular Biology. John Wiley and Sons, 1994
- **Hale ; Marham.** The Harper Collins Dictionary of Biology. Harper Perennial, 1991
- Immunol. Rev. 1987, vol. 98, 187
- **Smith ; Waterman.** *Adv. Appl. Math.,* 1981, vol. 2, 482
- **Needleman ; Wunsch.** *J. Mol. Biol.,* 1970, vol. 48, 443
- **Pearson ; Lipman.** *Proc. Natl. Acad. Sci. USA,* 1988, vol. 85, 2444
- **Devereux et al.** *Nucl. Acid Res.,* 1984, vol. 12, 387-395
- **Feng ; Doolittle.** *J. Mol. Evol.,* 1987, vol. 35, 351-360
- **Higgins ; Sharp.** *CABIOS,* 1989, vol. 5, 151-153
- **Altschul et al.** *J. Mol. Biol.,* 1990, vol. 215, 403-410
- **Karlin et al.** *Proc. Natl. Acad. Sci. USA,* 1993, vol. 90, 5873-5787
- **Altschul et al.** *Meth. Enzymol.,* 1996, vol. 266, 460-480
- **Henikoff ; Henikoff.** *Proc. Natl. Acad. Sci. USA,* 1989, vol. 89, 10915
- **Henikoff et al.** *Proc. Natl. Acad Sci. USA,* 1989, vol. 89, 10915
- **Karin et al.** *Proc. Natl Acad Sci USA,* 1993, vol. 90, 5873
- **Higgins et al.** *Gene,* 1988, vol. 73, 237-244
- **Pearson et al.** *Proc. Natl. Acad. Sci. USA,* 1988, vol. 85, 2444-2448
- **Chang ; Cohen.** *Mol. Gen. Genet.,* 1979, vol. 168, 111-115
- **Smith et al.** *Appl. Env. Microbiol.,* 1986, vol. 51, 634
- **Ferrari et al.** Bacillus. Plenum Publishing Corporation, 1989, 57-52
- **Sambrook et al.** Molecular Cloning: A Laboratory Manual. Cold Spring Harbor Laboratory Press, 1989, 16.7-16.8
- **Schmike et al.** *Science,* 1978, vol. 202, 1051
- **Kacian et al.** *Proc. Natl. Acad. Sci. USA,* 1972, vol. 69, 3038
- **Chamberlin et al.** *Nature,* 1970, vol. 228, 227
- **Wu ; Wallace.** *Genomics,* 1989, vol. 4, 560
- **Stemmer.** *Proc. Natl. Acad. Sci. USA,* 1994, vol. 91, 10747
- **Patten et al.** *Curr. Op. Biotechnol.,* 1997, vol. 8, 724
- **Kuchner ; Arnold.** *Trends Biotechnol.,* 1997, vol. 15, 523
- **Moore et al.** *J. Mol. Biol.,* 1997, vol. 272, 336
- **Zhao et al.** *Nature Biotechnol.,* 1998, vol. 16, 258
- **Giver et al.** *Proc. Nat'l Acad. Sci. USA,* 1998, vol. 95, 12809
- **Harayama.** *Trends BiotechnoL,* 1998, vol. 16, 76
- **Lin et al.** *Biotechnol., Prog.,* 1999, vol. 15, 467
- **Sun.** *Comput. Biol.,* 1999, vol. 6, 77
- **Taurog et al.** *Immunol. Rev.,* 1999, vol. 169, 209-223
- **Maeji et al.** *J. Immunol. Meth.,* 1990, vol. 134, 23-33
- **Mori et al.** *Transplant.,* 1997, vol. 64, 1017-1027
- **Marsh et al.** HLA Facts Book. The, Academic Press, 2000, 398